(19) Europäisches Patentamt
European Patent Office
Office européen des brevets

(11) Publication number: **0 200 362 B1**

(12) **EUROPEAN PATENT SPECIFICATION**

(45) Date of publication of patent specification: **20.01.93**

(51) Int. Cl.[5]: **C12Q 1/68**, C12P 19/34, C12N 15/10, //C07H21/00

(21) Application number: **86302298.4**

(22) Date of filing: **27.03.86**

Divisional application 92201243.0 filed on 27/03/86.

(54) **Process for amplifying, detecting, and/or cloning nucleic acid sequences.**

(30) Priority: **28.03.85 US 716975**
**25.10.85 US 791308**
**07.02.86 US 828144**

(43) Date of publication of application:
**10.12.86 Bulletin 86/45**

(45) Publication of the grant of the patent:
**20.01.93 Bulletin 93/03**

(84) Designated Contracting States:
**AT BE CH DE FR GB IT LI LU NL SE**

(56) References cited:
**EP-A- 0 086 548**
**EP-A- 0 138 242**
**EP-A- 0 139 501**
**EP-A- 0 155 188**
**US-A- 4 351 901**

**CHEMICAL ABSTRACTS, vol. 92, no. 9, 3rd March 1980, page 302, abstract no. 72232z, Columbus, Ohio, US; A.J. CATON et al.: "New procedure for the production of influenza virus-specific double-stranded DNA's", & NUCLEIC ACIDS RES. 1979, 7(6), 1445-56**

(73) Proprietor: **F. HOFFMANN-LA ROCHE AG**
**Postfach 3255**
**CH-4002 Basel(CH)**

(72) Inventor: **Mullis, Kary Banks**
**447 Beloit Avenue**
**Kensington California 94708(US)**
Inventor: **Arnheim, Norman**
**22322 De Kalb**
**Woodland Hills California 91364(US)**
Inventor: **Saiki, Randall Keichi**
**320-39th Street**
**Richmond California 94805(US)**
Inventor: **Erlich, Henry Anthony**
**3936 Rhoda Avenue**
**Oakland California 94602(US)**
Inventor: **Horn, Glenn Thomas**
**3 Admiral Drive F370**
**Emeryville California, 94608(US)**
Inventor: **Scharf, Stephen Joel**
**2028 1/2 Francisco Street**
**Berkeley California 94709(US)**

**EP 0 200 362 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**Kleppe et al (1971), J. Mol. Biol., 56, pages 341-361**

(74) Representative: **Bizley, Richard Edward**
**HEPWORTH LAWRENCE BRYER & BIZLEY**
**2nd Floor Gate House South West Gate**
**Harlow, Essex CM20 1JN(GB)**

## Description

The present invention relates to a process for amplifying existing nucleic acid sequences if they are present in a test sample and detecting them if present by using a probe. More specifically, it relates to a process for producing any particular nucleic acid sequence from a given sequence of DNA or RNA in amounts which are large compared to the amount initially present so as to facilitate detection of the sequences. The DNA or RNA may be single- or double-stranded, and may be a relatively pure species or a component of a mixture of nucleic acids. The process of the invention utilizes a repetitive reaction to accomplish the amplification of the desired nucleic acid sequence.

For diagnostic applications in particular, the target nucleic acid sequence may be only a small portion of the DNA or RNA in question, so that it may be difficult to detect its presence using nonisotopically labeled or end-labeled oligonucleotide probes. Much effort is being expended in increasing the sensitivity of the probe detection systems, but little research has been conducted on amplifying the target sequence so that it is present in quantities sufficient to be readily detectable using currently available methods.

Several methods have been described in the literature for the synthesis of nucleic acids de novo or from an existing sequence. These methods are capable of producing large amounts of a given nucleic acid of completely specified sequence.

One known method for synthesizing nucleic acids de novo involves the organic synthesis of a nucleic acid from nucleoside derivatives. This synthesis may be performed in solution or on a solid support. One type of organic synthesis is the phosphotriester method, which has been utilized to prepare gene fragments or short genes. In the phosphotriester method, oligonucleotides are prepared which can then be joined together to form longer nucleic acids. For a description of this method, see Narang, S.A., et al., Meth. Enzymol., 68, 90 (1979) and U.S. Patent No. 4,356,270. The patent describes the synthesis and cloning of the somatostatin gene.

A second type of organic synthesis is the phosphodiester method, which has been utilized to prepare a tRNA gene. See Brown, E.L., et al., Meth. Enzymol., 68, 109 (1979) for a description of this method. As in the phosphotriester method, the phosphodiester method involves synthesis of oligonucleotides which are subsequently joined together to form the desired nucleic acid.

Although the above processes for de novo synthesis may be utilized to synthesize long strands of nucleic acid, they are not very practical to use for the synthesis of large amounts of a nucleic acid. Both processes are laborious and time-consuming, require expensive equipment and reagents, and have a low overall efficiency. The low overall efficiency may be caused by the inefficiencies of the synthesis of the oligonucleotides and of the joining reactions. In the synthesis of a long nucleic acid, or even in the synthesis of a large amount of a shorter nucleic acid, many oligonucleotides would need to be synthesized and many joining reactions would be required. Consequently, these methods would not be practical for synthesizing large amounts of any desired nucleic acid.

Methods also exist for producing nucleic acids in large amounts from small amounts of the initial existing nucleic acid. These methods involve the cloning of a nucleic acid in the appropriate host system, where the desired nucleic acid is inserted into an appropriate vector which is used to transform the host. When the host is cultured the vector is replicated, and hence more copies of the desired nucleic acid are produced. For a brief description of subcloning nucleic acid fragements, see Maniatis, T., et al., Molecular Cloning: A Laboratory Manual, Cold Spring Harbor Laboratory, pp. 390-401 (1982). See also the techniques described in U.S. Patent Nos. 4,416,988 and 4,403,036.

A third method for synthesizing nucleic acids, described in U.S. Patent No. 4,293,652, is a hybrid of the above-described organic synthesis and molecular cloning methods. In this process, the appropriate number of oligonucleotides to make up the desired nucleic acid sequence is organically synthesized and inserted sequentially into a vector which is amplified by growth prior to each succeeding insertion.

The present invention bears some similarity to the molecular cloning method; however, it does not involve the propagation of any organism and thereby avoids the possible hazards or inconvenience which this entails. The present invention also does not require synthesis of nucleic acid sequences unrelated to the desired sequence, and thereby the present invention obviates the need for extensive purification of the product from a complicated biological mixture.

In J.Mol.Biol, 56 (1971), 341-361, Kleppe et al discuss primer extension reactions using templates corresponding to portions of a tRNA gene, in which reactions the primers are used are complementary to substantial parts of corresponding templates and are extended therealong thereby to provide duplex DNAs. These template copying reactions, involving simple primer extension, are termed "repair replication" by the authors. The final paragraph of the article theorises that if duplex DNA denaturation is effected in the presence of appropriate primers, two structures consisting of a full length template strand complexed to a

primer could be produced upon cooling, and repair replication achieved by adding DNA polymerase. The paragraph suggests that this process could be repeated. However, there is no detailed explanation of the precise techniques to be carried out, nor any discussion of which primers are "appropriate", and the possibility of a problem of template renaturation (reforming a duplex) is discussed with the suggestion that, if necessary, strand separation would have to be resorted to with subsequent repair replication.

The present invention resides in a process for amplifying one or more specific nucleic acid sequences present in a nucleic acid or mixture thereof using primers and agents for polymerization and then detecting the amplified sequence. The extension product of one primer when hybridized to the other becomes a template for the production of the desired specific nucleic acid sequence, and vice versa, and the process is repeated as often as is necessary to produce the desired amount of the sequence. This method is expected to be more efficient than the methods described above for producing large amounts of nucleic acid from a target sequence and to produce such nucleic acid in a comparatively short period of time. The present method is especially useful for amplifying rare species of nucleic acid present in a mixture of nucleic acids for effective detection of such species.

More specifically, the present invention provides a process for detecting the presence or absence of at least one specific double-stranded nucleic acid sequence in a sample, or distinguishing between two different double-stranded nucleic acid sequences in said sample, which process comprises first exponentially amplifying the specific sequence or sequences (if present) by the following steps, and then detecting the thus-amplified sequence or sequences (if present):

(a) separating the nucleic acid strands in the sample and treating the sample with a molar excess of a pair of oligonucleotide primers for each different specific sequence being detected, one primer for each strand, under hybridizing conditions and in the presence of an inducing agent for polymerization and the different nucleoside triphosphates such that for each of said strands an extension product of the respective primer is synthesized which is complementary to the strand, wherein said primers are selected so that each is substantially complementary to one end of the sequence to be amplified on one of the strands such that the extension product synthesized from one primer, when it is separated from its complement, can serve as a template for synthesis of an extension product of the other primer of the pair;

(b) treating the sample resulting from (a) under denaturing conditions to separate the primer extension products from their templates;

(c) treating as in (a) the sample resulting from (b) with oligonucleotide primers such that a primer extension product is synthesized using each of the single strands produced in step (b) as a template; and, if desired,

(d) repeating steps (b) and (c) at least once;

whereby exponential amplification of the nucleic acid sequence or sequences, if present, results thus permitting detection thereof; and, if desired,

(e) adding to the product of step (c) or (d) a labelled oligonucleotide probe capable of hybridizing to said sequence to be detected; and

(f) determining whether said hybridization has occurred.

The invention further provides a process wherein a single-stranded nucleic acid sequence which it is desired to detect is first treated under hybirdizing conditions with a primer, inducing agent for polymerization and the different nucleotides, to form a complementary strand thereto thereby to provide a starting double-stranded nucleic acid sequence and to permit detection of said single-stranded sequence.

The steps (a) to (c) may be conducted sequentially or simultaneously.

In another embodiment the invention relates to a kit for the detection of at least one specific nucleic acid sequence in a sample, which kit comprises, in packaged form, a multicontainer unit having:

(a) each of two oligonucleotide primers for each different sequence to be detected, wherein

(i) if the specific nucleic acid sequence to be detected is single-stranded one primer is substantially complementary to one end of the strand so that an extension product of said one primer formed under hybridizing conditions and in the presence of an inducing agent for polymerization and the different nucleoside triphosphates is substantially complementary to said strand, and the other primer is substantially complementary to one end of said extension product and can be used under hybridizing conditions and in the presence of an inducing agent for polymerization and the different nucleoside triphosphates to synthesize another extension product employing said extension product of said one primer as a template thereby providing a nucleic acid consisting of two strands; or

(ii) if the specific nucleic acid sequence to be detected is double-stranded the primers are such that each is substantially complementary to one end of one of the strands and an extension product synthesized from each primer using its complement strand as a template under hybridizing conditions

and in the presence of an inducing agent for polymerization and the different nucleoside triphosphates, when separated from its complement, can serve as a template for synthesis of an extension product of the other primer under hybridizing conditions and in the presence of an inducing agent for polymerization and the different nucleoside triphosphates;

(b) an agent for polymerization;

(c) each of the different nucleoside triphosphates;

(d) an oligonucleotide probe capable of hybridizing to said sequence if it is present in said sample; and

(e) means for detecting hybrids of said probe and said sequence.

In yet another embodiment, the invention provides a process for cloning into a vector a specific double-stranded nucleic acid sequence or sequences contained in a nucleic acid or a mixture of nucleic acids, which process comprises first amplifying said sequence or sequences, by the following steps:

(a) separating the nucleic acid strands and treating the nucleic acid(s) with a molar excess of a pair of oligonucleotide primers for each different specific sequence being amplified and cloned, one for each strand, under hybridizing conditions and in the presence of an inducing agent for polymerization and the different nucleoside triphosphates such that for each of said strands an extension product of each primer is synthesized which is complementary to its respective strand, wherein said primers are selected so that each is substantially complementary to one end of the sequence to be amplified on one of the strands such that the extension product synthesized from one primer, when it is separated from its complement, can serve as a template for synthesis of the extension product of the other primer of the pair and wherein said primers each contain a restriction site which is the same as or different from the restriction site(s) on the other primer(s);

(b) separating the primer extension products from the templates on which they were synthesized to produce single-stranded molecules;

(c) treating as in (a) the single-stranded molecules generated from step (b) with oligonucleotide primers such that a primer extension product is synthesized using each of the single strands produced in step (b) as a template; and, if desired,

(d) repeating steps (b) and (c) at least once;

whereby exponential amplification of the nucleic acid sequence or sequences results;

(e) adding to the product of step (c) or (d) a restriction enzyme for each of said restriction sites to obtain cleaved products in a restriction digest; and

(f) ligating the cleaved product(s) into one or more cloning vectors.

In yet another embodiment, the invention herein relates to a process for synthesizing a nucleic acid fragment from an existing double-stranded nucleic acid sequence having fewer nucleotides than the fragment being synthesized and two oligonucleotide primers, wherein the nucleic acid being synthesized is comprised of a left segment, a core segment and a right segment, and wherein the core segment represents at least substantially the nucleotide sequence of said existing nucleic acid sequence, and the right and left segments represent the nucleotide sequence present at one end of each of the two primers, the other ends of which are complementary or substantially complementary to ends of the single strands produced by separating the strands of said existing nucleic acid sequence, which process comprises:

(a) treating the strands of said existing fragment with a molar excess of two oligonucleotide primers, one for each of the strands, under hybridizing conditions and in the presence of an inducing agent for polymerization and the different nucleoside triphosphates such that for each strand an extension product of the respective primer is synthesized which is complementary to the strand, wherein said primers are selected so as each to be substantially complementary to one end of a strand of said existing sequence such that the extension product synthesized from one primer, when it is separated from its complement, can serve as a template for synthesis of an extension product of the other primer, and wherein each primer also contains a sequence of nucleotides which are not complementary to said existing sequence and which correspond to the two ends of the nucleic acid fragment being synthesized;

(b) separating the primer extension products from the templates on which they were synthesized to produce single-stranded molecules; and

(c) treating the single-stranded molecules generated from step (b) with the primers of step (a) under conditions such that a primer extension product is synthesized using each of the single strands produced in step (b) as a template so as to produce two intermediate double-stranded nucleic acid molecules, into each of which has been incorporated the nucleotide sequence present at one end of the oligonucleotide primers, and two full-length double-stranded nucleic acid molecules, into each of which has been incorporated the nucleotide sequence present at one end of both of the oligonucleotide primers;

(d) repeating steps (b) and (c) for a sufficient number of times to produce the full-length double-stranded molecules in an effective amount.

The core fragment may be obtained by:

treating two oligonucleotides, each of which has at one end thereof a nucleotide sequence which is complementary to an end of the other oligonucleotide and which are non-complementary to each other at their other ends, with an agent for polymerization and the different nucleoside triphosphates under conditions such that an extension product of each oligonucleotide is synthesized which is complementary to the other of the resulting extension products.

Co-pending European Application No. 201184 discloses processes for amplifying nucleic acid sequences.

Figure 1 illustrates a 94 base pair length sequence of human β-globin desired to be amplified. The single base pair change which is associated with sickle cell anemia is depicted beneath the 94-mer.

Figure 2 illustrates a photograph of an ethidium bromide-stained polyacrylamide gel demonstrating amplification of the 94-mer contained in human wild-type DNA and in a plasmid containing a 1.9 kb BamHI fragment of the normal β-globin gene (designated pBR328:HbA).

Figure 3 illustrates a photograph of an ethidium bromide-stained polyacrylamide gel demonstrating amplification of any of the specific target 94-mer sequence present in pBR328:HbA, a plasmid containing a 1.9 kb BamHI fragment of the sickle cell allele of β-globin (designated pBR328:HbS), pBR328:HbA where the sequence to be amplified is cleaved with MstII, and pBR328:HbS where the sequence to be amplified has been treated but not cleaved with MstII.

Figure 4 illustrates in detail the steps and products of the polymerase chain reaction for amplification of the desired 94-mer sequence of human β-globin for three cycles using two oligonucleotide primers.

Figure 5 represents a photograph of an ethidium bromide-stained polyacrylamide gel demonstrating amplification after four cycles of a 240-mer sequence in pBR328:HbA, where the aliquots are digested with NcoI (Lane 3), MstII (Lane 4) or HinfI (Lane 5). Lane 1 is the molecular weight standard and Lane 2 contains the intact 240-bp product.

Figure 6 illustrates the sequence of the normal ($\beta^A$) and sickle cell ($\beta^S$) β-globin genes in the region of the DdeI and HinfI restriction sites, where the single lines for $\beta^A$ mark the position of the DdeI site (CTGAG) and the double bars for $\beta^A$ and $\beta^S$ mark the position of the HinfI site (GACTC).

Figure 7 illustrates the results of sequential digestion of normal β-globin using a 40-mer probe and DdeI followed by HinfI restriction enzymes.

Figure 8 illustrates the results of sequential digestion of sickle β-globin using the same 40-mer probe as in Figure 7 and DdeI followed by HinfI restriction enzymes.

Figure 9 illustrates a photograph of an ethidium bromide-stained polyacrylamide gel demonstrating the use of the same 40-mer probe as in Figure 7 to specifically characterize the beta-globin alleles present in samples of whole human DNA which have been subjected to amplification, hybridization with the probe, and sequential digestion with DdeI and HinfI.

Figure 10 illustrates a photograph of a 6% NuSieve agarose gel visualized using ethidium bromide and UV light. This photograph demonstrates amplification of a sub-fragment of 110-bp amplification product which sub-fragment is an inner nested set within the 110-bp fragment.

The term "oligonucleotide" as used herein in referring to primers, probes, oligomer fragments to be detected, oligomer controls and unlabeled blocking oligomers is defined as a molecule comprised of two or more deoxyribonucleotides or ribonucleotides, preferably more than three. Its exact size will depend on many factors, which in turn depend on the ultimate function or use of the oligonucleotide.

The term "primer" as used herein refers to an oligonucleotide whether occurring naturally as in a purified restriction digest or produced synthetically, which is capable of acting as a point of initiation of synthesis when placed under conditions in which synthesis of a primer extension product which is complementary to a nucleic acid strand is induced, i.e., in the presence of nucleotides and an agent for polymerization such as DNA polymerase and at a suitable temperature and pH. The primer is preferably single stranded for maximum efficiency in amplification, but may alternatively be double stranded. If double stranded, the primer is first treated to separate its strands before being used to prepare extension products. Preferably, the primer is an oligodeoxyribonucleotide. The primer must be sufficiently long to prime the synthesis of extension products in the presence of the agent for polymerization. The exact lengths of the primers will depend on many factors, including temperature and source of primer. For example, depending on the complexity of the target sequence, the oligonucleotide primer typically contains 15-25 or more nucleotides, although it may contain fewer nucleotides. Short primer molecules generally require cooler temperatures to form sufficiently stable hybrid complexes with template.

The primers herein are selected to be "substantially" complementary to the different strands of each specific sequence to be amplified. This means that the primers must be sufficiently complementary to hybridize with their respective strands. Therefore, the primer sequence need not reflect the exact sequence

of the template. For example, a non-complementary nucleotide fragment may be attached to the 5' end of the primer, with the remainder of the primer sequence being complementary to the strand. Alternatively, non-complementary bases or longer sequences can be interspersed into the primer, provided that the primer sequence has sufficient complementarity with the sequence of the strand to be amplified to hybridize therewith and thereby form a template for synthesis of the extension product of the other primer.

As used herein, the terms "restriction endonucleases" and "restriction enzymes" refer to bacterial enzymes each of which cut double-stranded DNA at or near a specific nucleotide sequence.

As used herein, the term "DNA polymorphism" refers to the condition in which two or more different nucleotide sequences can exist at a particular site in DNA.

The term "restriction fragment length polymorphism" ("RFLP") refers to the differences among individuals in the lengths of restriction fragments formed by digestion with a particular restriction endonuclease.

The present invention is directed to a process for amplifying any one or more desired specific nucleic acid sequences suspected of being in a nucleic acid. Because large amounts of a specific sequence may be produced by this process, the present invention may be used for improving the efficiency of cloning DNA or messenger RNA and for amplifying a target sequence to facilitate detection thereof.

In general, the present process involves a chain reaction for producing, in exponential quantities relative to the number of reaction steps involved, at least one specific nucleic acid sequence given (a) that the ends of the required sequence are known in sufficient detail that oligonucleotides can be synthesized which will hybridize to them, and (b) that a small amount of the sequence is available to initiate the chain reaction. The product of the chain reaction will be a discrete nucleic acid duplex with termini corresponding to the ends of the specific primers employed.

Any source of nucleic acid, in purified or nonpurified form, can be utilized as the starting nucleic acid or acids, provided it is suspected of containing the specific nucleic acid sequence desired. Thus, the process may employ, for example, DNA or RNA, including messenger RNA, which DNA or RNA may be single stranded or double stranded. In addition, a DNA-RNA hybrid which contains one strand of each may be utilized. A mixture of any of these nucleic acids may also be employed, or the nucleic acids produced from a previous amplification reaction herein using the same or different primers may be so utilized. The specific nucleic acid sequence to be amplified may be only a fraction of a larger molecule or can be present initially as a discrete molecule, so that the specific sequence constitutes the entire nucleic acid. It is not necessary that the sequence to be amplified be present initially in a pure form; it may be a minor fraction of a complex mixture, such as a portion of the $\beta$-globin gene contained in whole human DNA or a portion of nucleic acid sequence due to a particular microorganism which organism might constitute only a very minor fraction of a particular biological sample. The starting nucleic acid may contain more than one desired specific nucleic acid sequence which may be the same or different. Therefore, the present process is useful not only for producing large amounts of one specific nucleic acid sequence, but also for amplifying simultaneously more than one different specific nucleic acid sequence located on the same or different nucleic acid molecules.

The nucleic acid or acids may be obtained from any source, for example, from plasmids such as pBR322, from cloned DNA or RNA, or from natural DNA or RNA from any source, including bacterial, yeast, viruses, and higher organisms such as plants or animals. DNA or RNA may be extracted from blood, tissue material such as chorionic villi or amniotic cells by a variety of techniques such as that described by Maniatis et al., Molecular Cloning (1982), 280-281.

Any specific nucleic acid sequence can be produced by the present process. It is only necessary that a sufficient number of bases at both ends of the sequence be known in sufficient detail so that two oligonucleotide primers can be prepared which will hybridize to different strands of the desired sequence and at relative positions along the sequence such that an extension product synthesized from one primer, when it is separated from its template (complement), can serve as a template for extension of the other primer into a nucleic acid of defined length. The greater the knowledge about the bases at both ends of the sequence, the greater can be the specificity of the primers for the target nucleic acid sequence, and thus the greater the efficiency of the process. It will be understood that the word primer as used hereinafter may refer to more than one primer, particularly in the case where there is some ambiguity in the information regarding the terminal sequence(s) of the fragment to be amplified. For instance, in the case where a nucleic acid sequence is inferred from protein sequence information a collection of primers containing sequences representing all possible codon variations based on degeneracy of the genetic code will be used for each strand. One primer from this collection will be homologous with the end of the desired sequence to be amplified.

The oligonucleotide primers may be prepared using any suitable method, such as, for example, the phosphotriester and phosphodiester methods described above, or automated embodiments thereof. In one

such automated embodiment diethylphosphoramidites are used as starting materials and may be synthesized as described by Beaucage et al., Tetrahedron Letters (1981), 22:1859-1862. One method for synthesizing oligonucleotides on a modified solid support is described in U.S. Patent No. 4,458,066. It is also possible to use a primer which has been isolated from a biological source (such as a restriction endonuclease digest).

The specific nucleic acid sequence is produced by using the nucleic acid containing that sequence as a template. If the nucleic acid contains two strands, it is necessary to separate the strands of the nucleic acid before it can be used as the template, either as a separate step or simultaneously with the synthesis of the primer extension products. This strand separation can be accomplished by any suitable denaturing methods including physical, chemical or enzymatic means. One physical method of separating the strands of the nucleic acid involves heating the nucleic acid until it is completely (>99%) denatured. Typical heat denaturation may involve temperatures ranging from about 80 to 105° C for times ranging from about 1 to 10 minutes. Strand separation may also be induced by an enzyme from the class of enzymes known as helicases or the enzyme RecA, which has helicase activity and in the presence of riboATP is known to denature DNA. The reaction conditions suitable for separating the strands of nucleic acids with helicases are described by Kuhn Hoffmann-Berling, CSH-Quantitative Biology, 43:63 (1978), and techniques for using RecA are reviewed in C. Radding, Ann. Rev. Genetics, 16:405-37 (1982).

If the original nucleic acid containing the sequence to be amplified is single stranded, its complement is synthesized by adding one or two oligonucleotide primers thereto. If an appropriate single primer is added, a primer extension product is synthesized in the presence of the primer, an agent for polymerization and the four nucleotides described below. The product will be partially complementary to the single-stranded nucleic acid and will hybridize with the nucleic acid strand to form a duplex of unequal length strands that may then be separated into single strands as described above to produce two single separated complementary strands. Alternatively, two appropriate primers may be added to the single-stranded nucleic acid and the reaction carried out.

If the original nucleic acid constitutes the sequence to be amplified, the primer extension product(s) produced will be completely complementary to the strands of the original nucleic acid and will hybridize therewith to form a duplex of equal length strands to be separated into single-stranded molecules.

When the complementary strands of the nucleic acid or acids are separated, whether the nucleic acid was originally double or single stranded, the strands are ready to be used as a template for the synthesis of additional nucleic acid strands. This synthesis can be performed using any suitable method. Generally it occurs in a buffered aqueous solution, preferably at a pH of 7-9, most preferably about 8. Preferably, a molar excess (for cloned nucleic acid, usually about 1000:1 primer:template, and for genomic nucleic acid, usually about $10^6$:1 primer:template) of the two oligonucleotide primers is added to the buffer containing the separated template strands. It is understood, however, that the amount of complementary strand may not be known if the process herein is used for diagnostic applications, so that the amount of primer relative to the amount of complementary strand cannot be determined with certainty. As a practical matter, however, the amount of primer added will generally be in molar excess over the amount of complementary strand (template) when the sequence to be amplified is contained in a mixture of complicated long-chain nucleic acid strands. A large molar excess is preferred to improve the efficiency of the process.

The deoxyribonucleoside triphosphates dATP, dCTP, dGTP and TTP are also added to the synthesis mixture in adequate amounts and the resulting solution is heated to about 90-100° C for from about 1 to 10 minutes, preferably from 1 to 4 minutes. After this heating period the solution is allowed to cool to from 20-40° C, which is preferable for the primer hybridization. To the cooled mixture is added an agent for polymerization, and the reaction is allowed to occur under conditions known in the art. This synthesis reaction may occur at from room temperature up to a temperature above which the agent for polymerization no longer functions efficiently. Thus, for example, if DNA polymerase is used as the agent for polymerization, the temperature is generally no greater than about 45° C. Preferably an amount of dimethylsulfoxide (DMSO) is present which is effective in detection of the signal or the temperature is 35-40° C. Most preferably, 5-10% by volume DMSO is present and the temperature is 35-40° C. For certain applications, where the sequences to be amplified are over 110 base pair fragments, such as the HLA DQ-$\alpha$ or -$\beta$ genes, an effective amount (e.g., 10% by volume) of DMSO is added to the amplification mixture, and the reaction is carried at 35-40° C, to obtain detectable results or to enable cloning.

The agent for polymerization may be any compound or system which will function to accomplish the synthesis of primer extension products, including enzymes. Suitable enzymes for this purpose include, for example, E. coli DNA polymerase I, Klenow fragment of E. coli DNA polymerase I, T4 DNA polymerase, other available DNA polymerases, reverse transcriptase, and other enzymes, including heat-stable enzymes, which will facilitate combination of the nucleotides in the proper manner to form the primer extension

products which are complementary to each nucleic acid strand. Generally, the synthesis will be initiated at the 3' end of each primer and proceed in the 5' direction along the template strand, until synthesis terminates, producing molecules of different lengths. There may be agents, however, which initiate synthesis at the 5' end and proceed in the other direction, using the same process as described above.

The newly synthesized strand and its complementary nucleic acid strand form a double-stranded molecule which is used in the succeeding steps of the process. In the next step, the strands of the double-stranded molecule are separated using any of the procedures described about to provide single-stranded molecules.

New nucleic acid is synthesized on the single-stranded molecules. Additional inducing agent, nucleotides and primers may be added if necessary for the reaction to proceed under the conditions prescribed above. Again, the synthesis will be initiated at one end of the oligonucleotide primers and will proceed along the single strands of the template to produce additional nucleic acid. After this step, half of the extension product will consist of the specific nucleic acid sequence bounded by the two primers.

The steps of strand separation and extension product synthesis can be repeated as often as needed to produce the desired quantity of the specific nucleic acid sequence. As will be described in further detail below, the amount of the specific nucleic acid sequence produced will accumulate in an exponential fashion.

When it is desired to produce more than one specific nucleic acid sequence from the first nucleic acid or mixture of nucleic acids, the appropriate number of different oligonucleotide primers are utilized. For example, if two different specific nucleic acid sequences are to be produced, four primers are utilized. Two of the primers are specific for one of the specific nucleic acid sequences and the other two primers are specific for the second specific nucleic acid sequence. In this manner, each of the two different specific sequences can be produced exponentially by the present process.

The present invention can be performed in a step-wise fashion where after each step new reagents are added, or simultaneously, where all reagents are added at the initial step, or partially step-wise and partially simultaneous, where fresh reagent is added after a given number of steps. If a method of strand separation, such as heat, is employed which will inactivate the agent for polymerization, as in the case of a heat-labile enzyme, then it is necessary to replenish the agent for polymerization after every strand separation step. The simultaneous method may be utilized when a number of purified components, including an enzymatic means such as helicase, is used for the strand separation step. In the simultaneous procedure, the reaction mixture may contain, in addition to the nucleic acid strand(s) containing the desired sequence, the strand-separating enzyme (e.g., helicase), an appropriate energy source for the strand-separating enzyme, such as rATP, the four nucleotides, the oligonucleotide primers in molar excess, and the inducing agent, e.g., Klenow fragment of E. coli DNA polymerase I. If heat is used for denaturation in a simultaneous process, a heat-stable inducing agent such as a thermostable polymerase may be employed which will operate at an elevated temperature, preferably 65-90 °C depending on the inducing agent, at which temperature the nucleic acid will consist of single and double strands in equilibrium. For smaller lengths of nucleic acid, lower temperatures of about 50 °C may be employed. The upper temperature will depend on the temperature at which the enzyme will degrade or the temperature above which an insufficient level of primer hybridization will occur. Such a heat-stable enzyme is described, e.g., by A. S. Kaledin et al., Biokhimiya, 45, 644-651 (1980). Each step of the process will occur sequentially notwithstanding the initial presence of all the reagents. Additional materials may be added as necessary. After the appropriate length of time has passed to produce the desired amount of the specific nucleic acid sequence, the reaction may be halted by inactivating the enzymes in any known manner or separating the components of the reaction.

The process of the present invention may be conducted continuously. In one embodiment of an automated process, the reaction may be cycled through a denaturing region, a reagent addition region, and a reaction region. In another embodiment, the enzyme used for the synthesis of primer extension products can be immobilized in a column. The other reaction components can be continuously circulated by a pump through the column and a heating coil in series, thus the nucleic acids produced can be repeatedly denatured without inactivating the enzyme.

The present invention is demonstrated diagrammatically below where double-stranded DNA containing the desired sequence [S] comprised of complementary strands [$S^+$] and [$S^-$] is utilized as the nucleic acid. During the first and each subsequent reaction cycle extension of each oligonucleotide primer on the original template will produce one new ssDNA molecule product of indefinite length which terminates with only one of the primers. These products, hereafter referred to as "long products," will accumulate in a linear fashion: that is, the amount present after any number of cycles will be proportional to the number of cycles.

The long products thus produced will act as templates for one or the other of the oligonucleotide primers during subsequent cycles and will produce molecules of the desired sequence [$S^+$] or [$S^-$] These molecules will also function as templates for one or the other of the oligonucleotide primers, producing

further [$S^+$] and [$S^-$], and thus a chain reaction can be sustained which will result in the accumulation of [S] at an exponential rate relative to the number of cycles.

By-products formed by oligonucleotide hybridizations other than those intended are not self-catalytic (except in rare instances) and thus accumulate at a linear rate.

The specific sequence to be amplified, [S], can be depicted diagrammatically as:

```
[S+]     5'  AAAAAAAAAAXXXXXXXXXXCCCCCCCCCC  3'
[S-]     3'  TTTTTTTTTTYYYYYYYYYYGGGGGGGGGG  5'
```

The appropriate oligonucleotide primers would be:

```
Primer 1:        GGGGGGGGGG
Primer 2:        AAAAAAAAAA
```

so that if DNA containing [S]

```
....zzzzzzzzzzzzzzzzAAAAAAAAAAXXXXXXXXXXCCCCCCCCCCzzzzzzzzzzzzzzzz....
....zzzzzzzzzzzzzzzzTTTTTTTTTTYYYYYYYYYYGGGGGGGGGGzzzzzzzzzzzzzzzz....
```

is separated into single strands and its single strands are hybridized to Primers 1 and 2, the following extension reactions can be catalyzed by DNA polymerase in the presence of the four deoxyribonucleoside triphosphates:

```
                                     3'         5'
         extends <------------------ GGGGGGGGGG  Primer 1
....zzzzzzzzzzzzzzzzAAAAAAAAAAXXXXXXXXXXCCCCCCCCCCzzzzzzzzzzzzzzzz....
original template strand+

original template strand-
....zzzzzzzzzzzzzzzzTTTTTTTTTTYYYYYYYYYYGGGGGGGGGGzzzzzzzzzzzzzzzz....
      Primer 2       AAAAAAAAAA ----------> extends
                     5'        3'
```

On denaturation of the two duplexes formed, the products are:

```
3'                                                     5'
....zzzzzzzzzzzzzzzzTTTTTTTTTTYYYYYYYYYYGGGGGGGGGG
newly synthesized long product 1

5'                                                                  3'
....zzzzzzzzzzzzzzzzAAAAAAAAAAXXXXXXXXXXCCCCCCCCCCzzzzzzzzzzzzzzzz....
original template strand+

3'                                                                  5'
....zzzzzzzzzzzzzzzzTTTTTTTTTTYYYYYYYYYYGGGGGGGGGGzzzzzzzzzzzzzzzz....
original template strand-

               5'                                                   3'
                AAAAAAAAAAXXXXXXXXXXCCCCCCCCCCzzzzzzzzzzzzzzzz....
                newly synthesized long product 2
```

If these four strands are allowed to rehybridize with Primers 1 and 2 in the next cycle, agent for polymerization will catalyze the following reactions:

```
    Primer 2       5'  AAAAAAAAAA ---------------> extends to here

3'....zzzzzzzzzzzzzzzzzzTTTTTTTTTTYYYYYYYYYYGGGGGGGGGG 5'
newly synthesized long product 1

    extends <-------------------------- GGGGGGGGGG 5' Primer 1

5'....zzzzzzzzzzzzzzzAAAAAAAAAAXXXXXXXXXXCCCCCCCCCCzzzzzzzzzzzzzzz....3'
original template strand+


    Primer 2       5'  AAAAAAAAAA ------------------------> extends

3'....zzzzzzzzzzzzzzzzzzTTTTTTTTTTYYYYYYYYYYGGGGGGGGGGzzzzzzzzzz....5'
original template strand-

   extends to here <---------------- GGGGGGGGGG  5'  Primer 1

                 5' AAAAAAAAAAXXXXXXXXXXCCCCCCCCCCzzzzzzzzzzzzzzzz..3'
                    newly synthesized long product 2
```

If the strands of the above four duplexes are separated, the following strands are found:

```
                          5' AAAAAAAAAAXXXXXXXXXXCCCCCCCCCC 3'
                             newly synthesized [S+]

3'....zzzzzzzzzzzzzzzzzzzTTTTTTTTTTYYYYYYYYYYGGGGGGGGGG 5'
first cycle synthesized long product 1

3'....zzzzzzzzzzzzzzzzzzzTTTTTTTTTTYYYYYYYYYYGGGGGGGGGG 5'
newly synthesized long product 1

5'....zzzzzzzzzzzzzzzzzzzAAAAAAAAAAXXXXXXXXXXCCCCCCCCCCzzzzzzzzz....3'
original template strand+

                    5' AAAAAAAAAAXXXXXXXXXXCCCCCCCCCCzzzzzzzzzzzzzzzz...3'
                       newly synthesized long product 2

3'..zzzzzzzzzzzzzzzTTTTTTTTTTYYYYYYYYYYGGGGGGGGGGzzzzzzzzzzzzzzzz...5'
original template strand-

                    3' TTTTTTTTTTYYYYYYYYYYGGGGGGGGGG 5'
                       newly synthesized [S-]

                    5' AAAAAAAAAAXXXXXXXXXXCCCCCCCCCCzzzzzzzzzzzzzzz...3'
                       first cycle synthesized long product 2
```

It is seen that each strand which terminates with the oligonucleotide sequence of one primer and the complementary sequence of the other is the specific nucleic acid sequence [S] that is desired to be produced.

The steps of this process can be repeated indefinitely, being limited only by the amount of Primers 1 and 2, the agent for polymerization and nucleotides present. For detection, the number of cycles used is that required to produce a detectable signal, an amount which will depend, e.g., on the nature of the sample. For example, if the sample is pure or diluted, fewer cycles may be required than if it is a complex mixture. If the sample is human genomic DNA, preferably the number of cycles is from about 10-30.

The amount of original nucleic acid remains constant in the entire process, because it is not replicated. The amount of the long products increases linearly because they are produced only from the original nucleic acid. The amount of the specific sequence increases exponentially. Thus, the specific sequence will become the predominant species. This is illustrated in the following table, which indicates the relative amounts of the species theoretically present after n cycles, assuming 100% efficiency at each cycle:

Number of Double Strands
After 0 to n Cycles

| Cycle Number | Template | Long Products | Specific Sequence [S] |
|---|---|---|---|
| 0 | 1 | - | - |
| 1 | 1 | 1 | 0 |
| 2 | 1 | 2 | 1 |
| 3 | 1 | 3 | 4 |
| 5 | 1 | 5 | 26 |
| 10 | 1 | 10 | 1013 |
| 15 | 1 | 15 | 32,752 |
| 20 | 1 | 20 | 1,048,555 |
| n | 1 | n | $(2^n-n-1)$ |

When a single-stranded nucleic acid is utilized as the template, only one long product is formed per cycle.

The method herein may be utilized to clone a particular nucleic acid sequence for insertion into a suitable expression vector. The vector may then be used to transform an appropriate host organism to produce the gene product of the sequence by standard methods of recombinant DNA technology.

Normally, such cloning would either involve direct ligation into a vector or the addition of oligonucleotide linkers followed by restriction enzyme cleavage. Both of these methods involve, however, the inefficient blunt-end ligation reaction. Also, neither technique would control for the orientation or multiplicity of insertion of the amplified product into the cloning vector.

The amplification process herein may yield a mixture of nucleic acids, resulting from the original template nucleic acid, the expected target amplified products, and various background non-target products. The amplified product can also be a mixture if the original template DNA contains multiple target sequences, such as in a heterozygous diploid genome or when there is a family of related genes.

The primers herein may be modified to assist the rapid and specific cloning of the mixture of DNAs produced by the amplification reaction. In such modification the same or different restriction sites are incorporated at the 5' ends of the primers to result in restriction sites at the two ends of the amplified product. When cut with the appropriate enzymes, the amplified product can then be easily inserted into plasmid or vial vectors and cloned. This cloning allows the analysis or expression of individual amplified products, not a mixture.

Although the same restriction site can be used for both primers, the use of different sites allows the insertion of the product into the vector with a specific orientation and suppresses multiple insertions as well as insertions arising from amplifications based on only one of the two primers. The specific orientation is useful when cloning into single-strand sequencing vectors, when single-strand hybridization probes are used, or when the cloned product is being expressed.

One method to prepare the primers is to choose a primer sequence which differs minimally from the target sequence. Regions in which each of the primers is to be located are screened for homology to restriction sites appropriate to the desired vector. For example, the target sequence "CAGTATCCGA..." differs by only one base from one containing a BamHI site. A primer sequence is chosen to match the target exactly at its 3' end, and to contain the altered sequence and restriction site near its 5' end (for example, CAGgATCCGA, where the lower case letter symbolizes a mismatch with the target sequence). This minimally altered sequence will not interfere with the ability of the primer to hybridize to the original target sequence and to initiate polymerization. After the first amplification cycle the primer is copied, becomes the target, and matches exactly with new primers. After the amplification process, the products are

cleaved with the appropriate restriction enzymes, optionally separated from inhibitors of ligation such as the nucleotide triphosphates and salts by passing over a desalting column or molecular weight chromatography column, and inserted by ligation into a cloning vector such as bacteriophage M13. The gene may then be sequenced and/or expressed using well known techniques.

The second method for preparing the primers involves taking the 3' end of the primers from the target sequence and adding the desired restriction site(s) to the 5' end of the primer. For the above example, a HindIII site could be added to make the sequence "cgaagcttCAGTATCCGA...", where lower case letters are as described above. The added bases would not contribute to the hybridization in the first cycle of amplification, but would match in subsequent cycles. The final amplified products are then cut with restriction enzyme(s) and cloned and expressed as described above. The gene being amplified may be, for example, human beta-hemoglobin or the human HLA DQ, DR or DP-$\alpha$ and -$\beta$ genes.

In addition, the process herein can be used for in vitro mutagenesis. The oligodeoxyribonucleotide primers need not be exactly complementary to the DNA sequence which is being amplified. It is only necessary that they be able to hybridize to the sequence sufficiently well to be extended by the polymerase enzyme or by whatever other inducing agent is employed. The product of a polymerase chain reaction wherein the primers employed are not exactly complementary to the original template will contain the sequence of the primer rather than the template, thereby introducing an in vitro mutation. In further cycles this mutation will be amplified with an undiminished efficiency because no further mispaired primings are required. The mutant thus produced may be inserted into an appropriate vector by standard molecular biological techniques and might confer mutant properties on this vector such as the potential for production of an altered protein.

The process of making an altered DNA sequence as described above could be repeated on the altered DNA using different primers so as to induce further sequence changes. In this way a series of mutated sequences could gradually be produced wherein each new addition to the series could differ from the last in a minor way, but from the original DNA source sequence in an increasingly major way. In this manner changes could be made ultimately which were not feasible in a single step due to the inability of a very seriously mismatched primer to function.

In addition, the primer can contain as part of its sequence a non-complementary sequence provided that a sufficient amount of the primer contains a sequence which is complementary to the strand to be amplified. For example, a nucleotide sequence which is not complementary to the template sequence (such as, e.g., a promoter, linker, coding sequence, etc.) may be attached at the 5' end of one or both of the primers, and thereby appended to the product of the amplification process. After the extension primer is added, sufficient cycles are run to achieve the desired amount of new template containing the non-complementary nucleotide insert. This allows production of large quantities of the combined fragments in a relatively short period of time (e.g., two hours or less) using a simple technique.

Moreover, the process herein may be used to synthesize a nucleic acid fragment from an existing nucleic acid fragment which is shorter than its product (called the core segment) using certain primers the 3' ends of which are complementary to or substantially complementary to the 3' ends of the single strands produced by separating the strands of the original shorter nucleic acid fragments, and the 5' ends of which primers contain sequence information to be appended to the core segment. This process comprises:

(a) treating the strands of said existing fragment with two oligonucleotide primers under conditions such that an extension product of each primer is synthesized which is complementary to each nucleic acid strand, wherein said primers are selected to as to be substantially complementary to the 3' end of each strand of said existing fragment such that the extension product synthesized from one primer, when it is separated from its complement, can serve as a template for synthesis of the extension product of the other primer, and wherein each primer contains, at its 5' end, a sequence of nucleotides which are not complementary to said existing fragment and which correspond to the two ends of the nucleic acid fragment being synthesized;

(b) separating the primer extension products from the templates on which they were synthesized to produce single-stranded molecules; and

(c) treating the single-stranded molecules generated from step (b) with the primers of step (a) under conditions such that a primer extension product is synthesized using each of the single strands produced in step (b) as a template so as to produce two intermediate double-stranded nucleic acid molecules, into each of which has been incorporated the nucleotide sequence present in the 5' end of one of the oligonucleotide primers, and two full-length double-stranded nucleic acid molecules, into each of which has been incorporated the nucleotide sequence present in the 5' ends of both of the oligonucleotide primers;

(d) repeating steps (b) and (c) for a sufficient number of times to produce the full-length double-stranded

molecules in an effective amount;

(e) treating the strands of the product of step (d) with two primers so as to lengthen the product of step (d) on both ends; and

(f) repeating steps (a)-(d) using the product of step (d) as the core fragment and two oligonucleotide primers which are complementary or substantially complementary to the 3' ends of the single strands produced by separating the strands of the product of step (d).

Steps (b) and (c) are repeated as often as necessary, usually at least 5 times, to produce the required amount of the full-length double-stranded product to synthesize the final product (i.e., the effective amount). In addition, the core segment may be obtained as the product of a previous amplification cycle. The product produced in step (d) may be purified before a new cycle of extension and amplification, or used directly by employing the reaction mixture containing the product.

If the 3' ends of the primers are not exactly complementary to the 3' ends of the single strands of the original shorter nucleic acid, the core fragment of the product will not be exactly the same as the sequence information resident in the original shorter nucleic acid. Therefore, mutants of the original nucleic acid may be made by using primers which are substantially complementary at their 3' ends to the 3' ends of the single strands of the original shorter nucleic acid.

If restriction site linkers are incorporated into the primers, then the amplified double-stranded products can be digested with the appropriate restriction enzymes and ligated directly into an M13 vector for rapid cloning and sequencing. The M13 plaques containing the specific amplified target sequences can be identified by hybridizing plaque lift filters with a probe specific for the target sequence.

The method herein may also be used to enable detection and/or characterization of specific nucleic acid sequences associated with infectious diseases, genetic disorders or cellular disorders such as cancer, e.g., oncogenes. Amplification is useful when the amount of nucleic acid available for analysis is very small, as, for example, in the prenatal diagnosis of sickle cell anemia using DNA obtained from fetal cells. Amplification is particularly useful if such an analysis is to be done on a small sample using non-radioactive detection techniques which may be inherently insensitive, or where radioactive techniques are being employed but where rapid detection is desirable.

For purposes of this invention genetic diseases may include specific deletions and/or mutations in genomic DNA from any organism, such as, e.g., sickle cell anemia, cystic fibrosis, α-thalassemia, β-thalassemia, and the like. Sickle cell anemia can be readily detected via oligomer restriction analysis or a RFLP-like analysis following amplification of the appropriate DNA sequence by the present method. α-Thalassemia can be detected by the absence of a sequence, and β-thalassemia can be detected by the presence of a polymorphic restriction site closely linked to a mutation which causes the disease.

All of these genetic diseases may be detected by amplifying the appropriate sequence and analyzing it by Southern blots without using radioactive probes. In such a process, for example, a small sample of DNA from, e.g., amniotic fluid containing a very low level of the desired sequence is amplified, cut with a restriction enzyme, and analyzed via a Southern blotting technique. The use of non-radioactive probes is facilitated by the high level of the amplified signal.

In another embodiment a small sample of DNA may be amplified to a convenient level and then a further cycle of extension reactions performed wherein nucleotide derivatives which are readily detectable (such as $^{32}\rho$-labeled or biotin labeled nucleoside triphosphates) are incorporated directly into the final DNA product, which may be analyzed by restriction and electrophoretic separation or any other appropriate method. An example of this technique in a model system is demonstrated in Figure 5.

In further embodiment, demonstrated in a model system in Figure 3, the nucleic acid may be exposed to a particular restriction endonuclease prior to amplification. Since a sequence which has been cut cannot be amplified, the appearance of an amplified fragment, despite prior restriction of the DNA sample, implies the absence of a site for the endonuclease within the amplified sequence. The presence or absence of an amplified sequence can be detected by an appropriate method.

A practical application of this technique can be illustrated by its use in facilitating the detection of sickle cell anemia via the oligomer restriction technique described herein and in Saiki et al., Biotechnology, 3:1008-1012 (1985). Sickle cell anemia is a hemoglobin disease which is caused by a single base pair change in the sixth codon of the β-globin gene. Figure 6 illustrates the sequences of normal and sickle cell β-globin genes in the region of their polymorphism, where the single bars mark the location of a DdeI site present only in the normal gene and where the double bars mark the location of a HinfI site which is non-polymorphic and thus present in both the normal and sickle cell alleles. Figure 7 illustrates the process of oligomer restriction of normal β-globin DNA using a probe spanning both restriction sites and labeled where the asterisk appears. (The probe is preferably labeled at the end which is fewer base pairs from the restriction site than the other end of the probe.) The DNA, amplified as provided herein, is denatured and

annealed to the labeled probe. The amplification may be carried out at elevated temperatures (35-40 °C) in the presence of dimethyl sulfoxide to minimize formation of secondary structure. The enzyme DdeI cleaves the DNA at the reformed DdeI site and generates a labeled octamer. Under the conditions used in the test the octamer is short enough to dissociate from the duplex. The subsequent addition of the enzyme HinfI has no effect on the now single-stranded octamer. Figure 8 illustrates the same process applied to the sickle cell allele of β-globin DNA. The enzyme DdeI cannot cleave the duplex formed by the amplified DNA and the labeled probe because of the A-A base pair mismatch. The enzyme HinfI, however, does restrict the hybrid and a labeled trimer is produced. In practice the method can diagnose the DNA of an individual as being either homozygous for the wild type, homozygous for the sickle type or a heterozygous carrier of the sickle cell trait, since a specific signal is associated with the presence of either allele. Use of this above-described method to amplify the pertinent sequence allows for a rapid analysis or a single copy gene using a probe with only a single $^{32}p$ label.

Various infectious diseases can be diagnosed by the presence in clinical samples of specific DNA sequences characteristic of the causative microorganism. These include bacteria, such as Salmonella, Chlamydia, Neisseria; viruses, such as the hepatitis viruses, and parasites, such as the Plasmodium responsible for malaria. U.S. Patent 4,358,535 issued to Falkow describes the use of specific DNA hybridization probes for the diagnosis of infectious diseases. A problem inherent in the Falkow procedure is that a relatively small number of pathogenic organisms may be present in a clinical sample from an infected patient and the DNA extracted from these may constitute only a very small fraction of the total DNA in the sample. Specific amplification of suspected sequences prior to immobilization and hybridization detection of the DNA samples could greatly improve the sensitivity and specificity of these procedures.

Routine clinical use of DNA probes for the diagnosis of infectious diseases would be simplified considerably if non-radioactively labeled probes could be employed as described in EP 63,879 to Ward. In this procedure biotin-containing DNA probes are detected by chromogenic enzymes linked to avidin or biotin-specific antibodies. This type of detection is convenient, but relatively insensitive. The combination of specific DNA amplification by the present method and the use of stably labeled probes could provide the convenience and sensitivity required to make the Falkow and Ward procedures useful in a routine clinical setting.

In addition, the probe may be a biotinylated probe in which the biotin is attached to a spacer arm of the formula:

$$-Y-(CH_2)_2-O-[(CH_2)_xO]_y-CH_2CH_2-\overset{H}{\overset{|}{N}}-$$

where Y is 0, NH or N-CHO, x is a number from 1 to 4, and y is a number from 2 to 4. The spacer arm is in turn attached to a psoralen moiety of the formula:

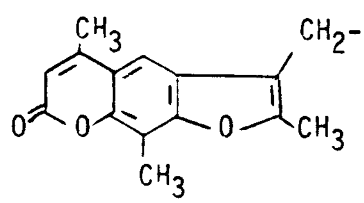
.

The psoralen moiety intercalates into and crosslinks a "gapped circle" probe as described by Courage-Tebbe et al., Biochim. Biophys. Acta, 697 (1982) 1-5, wherein the single-stranded hybridization region of the gapped circle spans the region contained in the primers.

The amplification process can also be utilized to produce sufficient quantities of DNA from a single copy human gene such that detection by a simple non-specific DNA stain such as ethidium bromide can be employed so as to make a DNA diagnosis directly.

In addition to detecting infectious diseases and pathological abnormalities in the genome of organisms, the process herein can also be used to detect DNA polymorphism which may not be associated with any pathological state.

The following examples are offered by way of illustration and are not intended to limit the invention in any manner. In these examples all percentages are by weight if for solids and by volume if for liquids, and

all temperatures are in degrees Celsius unless otherwise noted.

EXAMPLE 1

A 25 base pair sequence having the nucleotide sequence

5' CCTCGGCACCGTCACCCTGGATGCT 3'
3' GGAGCCGTGGCAGTGGGACCTACGA 5'

contained on a 47 base pair FokI restriction fragment of pBR322 obtainable from ATCC was prepared as follows. AFokI digest of pBR322 containing the 47-bp fragment was produced by digesting pBR322 with FokI in accordance with the conditions suggested by the supplier, New England Biolabs Inc. The primers which were utilized were 5' d(CCTCGGCACCG) 3' and 5' d(AGCATCCAGGGTG) 3', and were prepared using conventional techniques. The following ingredients were added to 33 µl of buffer which consisted of 25 mM potassium phosphate, 10 mM magnesium chloride and 100mM sodium chloride at pH 7.5: 2433 pmoles of each of the primers described above, 2.4 pmoles of the FokI digest of pBR322, 12 nmoles of dATP, 22 nmoles of dCTP, 19 nmoles of dGTP and 10 nmoles of TTP.

The mixture was heated to 85° C for five minutes and allowed to cool to ambient temperature. Five units of the Klenow fragment of E. coli DNA polymerase 1 were added and the temperature was maintained for 15 minutes. After that time, the mixture was again heated to 85° C for five minutes and allowed to cool. Five units of the Klenow fragment were again added and the reaction was carried out for 15 minutes. The heating, cooling and synthesis steps were repeated eleven more times.

After the final repetition, a 5 µl aliquot was removed from the reaction mixture. This was heated to 85° C for three minutes and allowed to cool to ambient temperature. 12.5 pmoles of a $\alpha$-$\rho^{32}$ -deoxycytidine triphosphate and 5 units of Klenow fragment were added and the reaction was allowed to proceed for 15 minutes. The labeled products were examined by polyacrylamide gel electrophoresis. The FokI digest was labeled in a similar fashion and served as a control and molecular weight markers. The only heavily labeled band visible after the 13 cycles was the intended 25 base pair sequence.

EXAMPLE 2

The desired sequence to be amplified was a 94 base pair sequence contained within the human beta-globin gene and spanning the MstII site involved in sickle cell anemia. The sequence has the nucleotide sequence shown in Figure 1.

1. Synthesis of Primers

The following two oligodeoxyribonucleotide primers were prepared by the method described below:

5' CACAGGGCAGTAACG 3' Primer A

and

5' TTTGCTTCTGACACA 3' Primer B

Automated Synthesis Procedures: The diethylphosphoramidites, synthesized according to Beaucage and Caruthers (Tetrahedron Letters (1981) 22:1859-1862) were sequentially condensed to a nucleoside derivatized controlled pore glass support using a Biosearch SAM-1. The procedure included detritylation with trichloroacetic acid in dichloromethane, condensation using benzotriazole as activating proton donor, and capping with acetic anhydride and dimethylaminopyridine in tetrahydrofuran and pyridine. Cycle time was approximately 30 minutes. Yields at each step were essentially quantitative and were determined by collection and spectroscopic examination of the dimethoxytrityl alcohol released during detritylation.

Oligodeoxyribonucleotide Deprotection and Purification Procedures: The solid support was removed from the column and exposed to 1 ml concentrated ammonium hydroxide at room temperature for four hours in a closed tube. The support was then removed by filtration and the solution containing the partially protected oligodeoxynucleotide was brought to 55° C for five hours. Ammonia was removed and the residue was applied to a preparative polyacrylamide gel. Electrophoresis was carried out at 30 volts/cm for 90

minutes after which the band containing the product was identified by UV shadowing of a fluorescent plate. The band was excised and eluted with 1 ml distilled water overnight at 4° C. This solution was applied to an Altech RP18 column and eluted with a 7-13% gradient of acetonitrile in 1% ammonium acetate buffer at pH 6.0. The elution was monitored by UV absorbance at 260 nm and the appropriate fraction collected, quantitated by UV absorbance in a fixed volume and evaporated to dryness at room temperature in a vacuum centrifuge.

Characterization of Oligodeoxyribonucleotides: Test aliquots of the purified oligonucleotides were $^{32}\rho$ labeled with polynucleotide kinase and $\gamma$-$^{32}\rho$-ATP. The labeled compounds were examined by autoradiography of 14-20% polyacrylamide gels after electrophoresis for 45 minutes at 50 volts/cm. This procedure verifies the molecular weight. Base composition was determined by digestion of the oligodeoxyribonucleotide to nucleosides by use of venom diesterase and bacterial alkaline phosphatase and subsequent separation and quantitation of the derived nucleosides using a reverse phase HPLC column and a 10% acetonitrile, 1% ammonium acetate mobile phase.

II. Source of DNA

A. Extraction of Whole Human Wild-Type DNA

Human genomic DNA homozygous for normal $\beta$-globin was extracted from the cell line Molt4 (obtained from Human Genetic Mutant Cell Repository and identified as Gm2219c) using the technique described by Stetler et al., Proc. Nat. Acad. Sci. (1982), 79:5966-5970.

B. Construction of Cloned Globin Genes

A 1.9 kb BamHI fragment of the normal $\beta$-globin gene was isolated from the cosmid pFCII and inserted into the BamHI site of pBR328 (Soberon, et al., Gene (1980) 9:287-305). This fragment, which encompasses the region that hybridizes to the synthetic 40-mer probe, includes the first and second exons, first intron, and 5' flanking sequences of the gene (Lawn et al., Cell (1978), 15:1157-1174). This clone was designated pBR328:HbA and deposited under ATCC No. 39,698 on May 25, 1984.

The corresponding 1.9 kb BamHI fragment of the sickle cell allele of $\beta$-globin was isolated from the cosmid pFC12 and cloned as described above. This clone was designated pBR328:HbS and deposited under ATCC No. 39,699 on May 25, 1984.

Each recombinant plasmid was transformed into and propagated in E. coli MM294 (ATCC No. 39,607).

C. Digestion of Cloned Globin Genes with MstII

A total of 100 $\mu$g each of pBR328:HbA and pBR328:HbS were individually digested with 20 units of MstII (New England Biolabs) for 16 hours at 37° C in 200 $\mu$l of 150 mM NaCl, 12 mM Tris HCl (pH 7.5), 12 mM $MgCl_2$, 1 mM dithiothreitol (DTT), and 100 $\mu$g/ml bovine serum albumin (BSA). The products are designated pBR328:HbA/MstII and pBR328:HbS/MstII, respectively.

III. Polymerase Chain Reaction

To 100 $\mu$l of buffer consisting of 60 mM sodium acetate, 30 mM Tris acetate and 10 mM magnesium acetate at pH 8.0 was added 2 $\mu$l of a solution containing 100 picomoles of Primer A (of the sequence d-(CACAGGCACTAACG)), 100 picomoles of Primer B (of the sequence d(TTTGCTTCTGACACA)) and 1000 picomoles each of dATP, dCTP, dGTP and TTP. In addition, one of the following sources of DNA described above was added:

10 $\mu$g whole human wild-type DNA (Reaction I)
0.1 picomole pBR328:HbA (Reaction II)
0.1 picomole pBR328:Hbs (Reaction III)
0.1 picomole pBR328:HbA/MstII (Reaction IV)
0.1 picomole pBR328:HbS/MstII (Reaction V)
No target DNA (Reaction VI)

Each resulting solution was heated to 100° C for four minutes and allowed to cool to room temperature for two minutes, whereupon 1 $\mu$l containing four units of Klenow fragment of E. coli DNA polymerase was added. Each reaction was allowed to proceed for 10 minutes, after which the cycle of adding the primers, nucleotides and DNA, heating, cooling, adding polymerase, and reacting was repeated nineteen times for

Reaction I and four times for Reactions II-VI.

Four microliter aliquots of Reactions I and II removed before the first cycle and after the last cycle of each reaction were applied to a 12% polyacrylamide gel 0.089 M in Tris-borate buffer at pH 8.3 and 2.5 mM in EDTA. The gel was electrophoresed at 25 volts/cm for four hours, transferred to a nylon membrane serving as solid phase support and probed with a 5'-$^{32}\rho$-labeled 40 bp synthetic fragment, prepared by standard techniques, of the sequence

5'd(TCCTGAGGAGAAGTCTGCCGTTACTGCCCTGTGGGGCAAG)3'

in 30% formamide, 3 x SSPE, 5 x Denhardt's, 5% sodium dodecyl sulfate at pH 7.4. Figure 2 is an autoradiograph of the probed nylon membrane for Reactions I and II. Lane 1 is 0.1 picomole of a 58-bp synthetic fragment control one strand of which is complementary to the above probe. Lane 2 is 4 µl of Reaction I prior to the first amplification cycle. Lane 3 is 4 µl of Reaction I after the 20th amplification cycle. Lane 4 is 4 µl of Reaction II after five amplification cycles. Lane 5 is a molecular weight standard consisting of a FokI (New England Biolabs) digest of pBR322 (New England Biolabs) labeled with alpha-$^{32}\rho$-dNTPs and polymerase. Lane 3 shows that after twenty cycles the reaction mixture I contained a large amount of the specific sequence of the proper molecular weight and no other detectable products. Reaction mixture II after five cycles also contained this product, as well as the starting nucleic acid and other products, as shown by Lane 4.

To 5.0 µl aliquots of Reactions II-VI after the fourth cycle were added 5 pmoles of each primer describved above. The solutions were heated to 100°C for four minutes and allowed to equilibrate to room temperature. Three pmoles each of alpha-$^{32}\rho$-dATP, alpha-$^{32}\rho$-dCTP, alpha-$^{32}\rho$-dGTP and alpha-$^{32}\rho$-TTP and four units of Klenow fragment were added. The reaction, in a final volume of 10 µl and at the salt concentrations given above, was allowed to proceed for 10 minutes. The polymerase activity was terminated by heating for 20 minutes at 60°C. Four µl aliquots of Reactions II-VI were loaded onto a 12% polyacrylamide gel 0.089 M in Tris-borate buffer at pH 8.3 and 2.5 mM in EDTA. The gel was electrophoresed at 25 volts/cm for four hours after which autoradiography was performed.

Figure 3 is an autoradiograph of the electrophoresis. Lane 1 is a molecular weight standard, Lane 2 is Reaction II, Lane 3 is Reaction III, Lane 4 is Reaction IV and Lane 5 is Reaction V. Another lane for Reaction VI with no DNA as control had no images in any of the lanes. It can be seen from the figure that the 94-bp fragment predicted from the target DNA was present only where intact β-globin DNA sequences were available for amplification, i.e., pBR328:HbA (Lane 2), pBR328:HbS (Lane 3) and pBR328:HbS/MstII (Lane 5). MstII digestion cuts pBR328:HbA in the 94-mer sequence rendering it incapable of being amplified, and the 94-mer band does not appear in Lane 4. In contrast, the 94-mer sequence in pBR328:HbS does not cut when the plasmid is digested with MstII and thus is available for amplification as shown in Lane 5.

Figure 4 illustrates the chain reaction for three cycles in amplifying the 94-bp sequence. PCO1 and PCO2 are Primers A and B. The numbers on the right indicate the cycles, whereas the numbers on the left indicates the cycle number in which a particular molecule was produced.

EXAMPLE 3

This example illustrates amplification of 110 bp sequence spanning the allelic MstII site in the human hemoglobin gene.

A total of 1.0 microgram whole human DNA, 100 picomoles d(ACACAACTGTGTTCACTAGC) and 100 picomoles d(CAACTTCATCCACGTTCACC) the primers having been prepared by the technique of Example 2, were dissolved in 100 µl of a solution which was:

1.5 mM in each of the four deoxyribonucleoside triphosphates
30 mM in Tris acetate buffer at pH 7.9
60 mM in sodium acetate
10 mM in magnesium acetate
0.25 mM in dithiothreitol

The solution was heated to 100°C for one minute and brought rapidly to 25°C for one minute, after which was added 2.5 units Klenow fragment of DNA polymerase. The polymerase reaction was allowed to proceed for two minutes at 25°C, after which the cycle of heating, cooling, adding Klenow, and reacting was repeated as often as desired.

With a 70% efficiency at each cycle, 15 cycles resulted in the synthesis of 1.4 femtomoles of the

desired 110 bp fragment of the β-globin gene.

EXAMPLE 4

This example illustrates amplification of a 240 bp sequence spanning the allelic MstII site in the human hemoglobin gene. This sequence contains NcoI, HinfI and MstII restriction sites.

To 100 μl of a mixture of 60 mM sodium acetate, 30 mM Tris acetate and 10 mM magnesium acetate at pH 8.0 containing 0.1 pmole pBR328:HbA was added 2 μl of Solution A containing:

100 pmoles d(GGTTGGCCAATCTACTCCCAGG) primer

100 pmoles d(TAACCTTGATACCAACCTGCCC) primer

1000 pmoles each of dATP, dCTP, dGTP and TTP

The two primers were prepared by the technique described in Example 2. The solution was heated to 100 °C for four minutes and allowed to cool in ambient air for two minutes, after which was added 1 μl containing four units Klenow fragment of E. coli DNA polymerase. The reaction was allowed to proceed for 10 minutes after which the cycle of solution A addition, heating, cooling, adding polymerase, and reacting was repeated three times. To a 5.0 μl aliquot of the reactions was added 5 picomoles of each oligonucleotide primer described above. The solution was heated to 100 °C for four minutes and allowed to come to ambient temperature, after which 3 picomoles each of the alpha-$^{32}\rho$-labeled deoxyribonucleoside triphosphates and 4 units Klenow fragment were added. The reaction, in a final volume of 10 μl and at the salt concentrations given above, was allowed to proceed for 10 minutes. The polymerase activity was terminated by heating for 20 minutes at 60 °C. Two μl aliquots were digested with NcoI, MstII, or HinfI and loaded onto a 12% polyacrylamide gel 0.089 M in Tris-borate buffer at pH 8.3 and 2.5 mM in EDTA. The gel was electrophoresed at 25 volts/cm for four hours and autoradiography was performed. Figure 5 illustrates the autoradiograph of the electrophoresis, where Lane 1 is the molecular weight standard, Lane 2 is without digestion with enzyme (240 bp intact), Lane 3 is digestion with NcoI (131 and 109 bp), Lane4 is digestion with MstII (149 and 91 bp), and Lane 5 is digestion with HinfI (144 and 96 bp). The autoradiograph is consistent with the amplification of the 240 bp sequence.

EXAMPLE 5

This example illustrates use of the process herein to detect sickle cell anemia by sequential digestion.

Synthesis and Phosphorylation of Oligodeoxyribonucleotides

A labeled DNA probe, RS06, of the sequence

5' *CTGACTCCTGAGGAGAAGTCTGCCGTTACTGCCCTGTGGG 3'

where * indicates the label, and an unlabeled blocking oligomer, RS10, of the sequence

3' GACAGAGGTCACCTCTTCAGACGGCAATGACGGGACACCC 5'

which has three base pair mismatches with RS06 were synthesized according to the procedures provided in Example 2(1) The probe RS06 was labeled by contacting five pmole thereof with 4 units of T4 polynucleotide kinase (New England Biolabs) and 50 pmole $\gamma^{-32}\rho$-ATP (New England Nuclear, about 7200 Ci/mmole) in a 40 μl reaction volume containing 70 mM Tris buffer (pH 7.6), 10 mM $MgCl_2$, 1.5 mM spermine, and 2.5 mM dithiothreitol for 90 minutes at 37 °C. The total volume was then adjusted to 100 μl with 25 mM EDTA and purified according to the procedure of Maniatis et al., Molecular Cloning (1982), 464-465 over a 1 ml Bio Gel P-4 spin dialysis column from BioRad equilibrated with Tris-EDTA (TE) buffer (10 mM Tris buffer, 0.1 mM EDTA, pH 8.0). The labeled probe was further purified by electrophoresis on a 18% polyacrylamide gel (19:1 acrylamide:BIS, BioRad) in Tris-boric acid-EDTA (TBE) buffer (89 mM Tris, 89 mM boric acid, 2.5 mM EDTA, pH 8.3) for 500 vhr. After localization by autoradiography, the portion of the gel containing the labeled probe was excised, crushed and eluted into 0.2 ml TE buffer overnight at 4 °C. TCA precipitation of the reaction product indicated that the specific activity was 4.9 Ci/mmole and the final concentration was 20 pmole/ml.

The unlabeled RS10 blocking oligomer was used at a concentration of 200 pmole/ml.

Isolation of Human Genomic DNA from Cell Lines

High molecular weight genomic DNA was isolated from the lymphoid cell lines Molt4, SC-1 and Gm2064 using essentially the method of Stetler et al., PNAS (1982), 79, 5966-5970 (for Molt4) and Maniatis et al., Molecular Cloning (1982), 280-281.

Molt4 (Human Mutant Cell Repository, GM2219C) is a T cell line homozygous for normal β-globin, and SC-1, deposited with ATCC on March 19, 1985, is an EBV-transformed B cell line homozygous for the sickle cell allele. GM2064 (Human Mutant Cell Repository, GM2064) was originally isolated from an individual homozygous for hereditary persistance of fetal hemoglobin (HPFH) and contains no beta- or delta-globin gene sequences. All cell lines were maintained in RPMI-1640 with 10% fetal calf serum.

Isolation of Human Genomic DNA from Clinical Blood Samples

A clinical blood sample designated CH12 from a known sickle cell carrier (AS) was obtained from Dr. Bertram Lubin of Children's Hospital in Oakland, California. Genomic DNA was prepared from the buffy coat fraction, which is composed primarily of peripheral blood lymphocytes, using a modification of the procedure described by Nunberg et al., Proc. Nat. Acad. Sci., 75, 5553-5556 (1978).

The cells were resuspended in 5 ml Tris-EDTA-NaCl (TEN) buffer (10 mM Tris buffer pH 8, 1 mM EDTA, 10 mM NaCl) and adjusted to 0.2 mg/ml proteinase K, 0.5% SDS, and incubated overnight at 37° C. Sodium perchlorate was then added to 0.7 M and the lysate gently shaken for 1-2 hours at room temperature. The lysate was extracted with 30 ml phenol/chloroform (1:1), then with 30 ml chloroform, and followed by ethanol precipitation of the nucleic acids. The pellet was resuspended in 2 ml of TE buffer and RNase A added to 0.005 mg/ml. After digestion for one hour at 37° C, the DNA was extracted once each with equal volumes of phenol, phenol/chloroform, and chloroform, and ethanol precipitated. The DNA was resuspended in 0.5 ml TE buffer and the concentration was determined by absorbance at 260 nm.

Polymerase Chain Reaction to Amplify Selectively β-Globin Sequences

Two micrograms of genomic DNA was amplified in an initial 100 µl reaction volume containing 10 mM Tris buffer (pH 7.5), 50 mM NaCl, 10 mM $MgCl_2$, 150 pmole of Primer A of the sequence d-(CACAGGGCACTAACG), and 150 pmole of Primer B of the sequence d(CTTTGCTTCTGACACA) and overlayed with about 100 µl mineral oil to prevent evaporation.

Each DNA sample underwent 15 cycles of amplification where one cycle is composed of three steps:

1) Denature in a heat block set a 95° C for two minutes.

2) Transfer immediately to a heat block set at 30° C for two minutes to allow primers and genomic DNA to anneal.

3) Add 2 µl of a solution containing 5 units of the Klenow fragment of E. coli DNA polymerase I (New England Biolabs), 1 nmole each of dATP, dCTP, dGTP and dTTP, in a buffer composed of 10mM Tris (pH 7.5), 50 mM NaCl, 10 mM $MgCl_2$, and 4 mM dithiothreitol. This extension reaction was allowed to proceed for 10 minutes at 30° C.

After the final cycle, the reaction was terminated by heating at 95° C for two minutes. The mineral oil was extracted with 0.2 ml of chloroform and discarded. The final reaction volume was 130 µl.

Hybridization/Digestion of Amplified Genomic DNA with Probes and DdeI/HinfI

Forty-five microliters of the amplified genomic DNA was ethanol precipitated and resuspended in an equal volume of TE buffer. Ten microliters (containing the pre-amplification equivalent of 154 ng of genomic DNA) was dispensed into a 1.5 ml Microfuge tube and 20 µl of TE buffer to a final volume of 30 µl. The sample was overlayed with mineral oil and denatured at 95° C for 10 minutes. Ten microliters of 0.6 M NaCl containing 0.02 pmole of labeled RS06 probe was added to the tube, mixed gently, and immediately transferred to a 56° C heater block for one hour. Four microliters of unlabeled RS10 blocking oligomer (0.8 pmole) was added and the hybridization continued for an additional 10 minutes at the same temperature. Five microliters of 60 mM $MgCl_2$/0.1% BSA and 1 µl of DdeI (10 units, New England Biolabs) were added and the reannealed DNA was digested for 30 minutes at 56° C. One microliter of HinfI (10 units, New England Biolabs) was then added and incubated for another 30 minutes. The reaction was stopped by the addition of 4 µl 75 mM EDTA and 6 µl tracking dye to a final volume of 61 µl.

The mineral oil was extracted with 0.2 ml chloroform, and 18 µl of the reaction mixture (45 ng genomic DNA) was loaded onto a 30% polyacrylamide mini-gel (19:1, Bio Rad) in a Hoeffer SE200 apparatus. The

gel was electrophoresed at approximately 300 volts for one hour until the bromphenol blue dye front migrated to 3.0 cm off-origin. The top 1.5 cm of the gel was removed and the remaining gel was exposed for four days with one intensification screen at -70 ° C.

Discussion of Photograph (Figure 9)

Each lane contains 45 ng of amplified genomic DNA. Lane A contains Molt4 DNA; Lane B, CH12; Lane C, SC-1; and Lane D, GM2064. Molt4 represents the genotype of a normal individual with two copies of the $\beta^A$ gene per cell (AA), CH12 is a clinical sample from a sickle cell carrier with one $\beta^A$ and one $\beta^S$ gene per cell (AS), and SC-1 represents the genotype of a sickle cell individual with two copies of the $\beta^S$ gene per cell (SS). GM2064, which contains no beta- or delta-globin sequences, is present as a negative control.

As seen in the photograph, the DdeI-cleaved, $\beta^A$-specific octamer is present only in those DNA's containing the $\beta^A$ gene (Lanes A and B) and the HinfI-cleaved, $\beta^S$-specific trimer is present only in those DNA's containing the $\beta^S$ (Lanes B and C). The presence of both trimer and octamer (Lane B) is diagnostic for a sickle cell carrier and is distinguishable from a normal individual (Lane A) with only octamer and a sickle cell afflicted individual (Lane C) with only trimer.

As a comparison, repeating the experiment described above using non-amplified genomic DNA revealed that the amplification increased the sensitivity of detection by at least 1000 fold.

EXAMPLE 6

This example illustrates direct detection of a totally unpurified single copy gene in whole human DNA on gels without the need for a labeled probe.

Using the technique described in Example 3, a 110-bp fragment from a sequence in the first exon of the beta-globin gene was amplified from 10 micrograms of whole human DNA after 20 cycles. This 110-bp fragment produced after 20 cycles was easily visualized on gels stained with ethidium bromide.

The sequence was not amplified when it was first cut with the restriction enzyme DdeI unless, as in the beta-globin S allele, the sequence does not contain the restriction site recognized by the enzyme.

EXAMPLE 7

A. A total of 100 fmoles pBR328 containing a 1.9 kb insert from the human beta-globin A allele, 50 nmoles each alpha-32P-dNTP at 500 Ci/mole, and 1 nmole of each of the primers used in Example 3 were dissolved in a solution containing 100 µl 30 mM Tris-acetate at pH 7.9, 60 mM sodium acetate, 100 mM dithiothreitol, and 10 mM magnesium acetate. This solution was brought to 100 ° C for two minutes and cooled to 25 ° C for one minute. A total of 1µl containing 4.5 units Klenow fragment of E. coli DNA polymerase I and 0.09 units inorganic pyrophosphatase was added to prevent the possible build-up of pyrophosphate in the reaction mixture, and the reaction was allowed to proceed for two minutes at 25 ° C, after which the cycle of heating, cooling, adding enzyme, and reacting was repeated nine times. Ten-µl aliquots were removed and added to 1 µl 600 mM EDTA after each synthesis cycle. Each was analyzed on a 14% polyacrylamide gel in 90 mM Tris-borate and 2.5 mM EDTA at pH 8.3 and 24 volts/cm for 2.5 hours. The completed gel was soaked for 20 minutes in the same buffer with the addition of 0.5 µg/ml ethidium bromide, washed with the original buffer, and photographed in UV light using a red filter.

The 110-bp fragment produced was excised from the gel under ultraviolet light and the incorporated $^{32}p$ counted by Cerenkov radiation. An attempt to fit the data to an equation of the form: pmoles/10 µl = 0.01 $[(1+y)^N\text{-}yN\text{-}1]$, where N represents the number of cycles and y the fractional yield per cycle, was optimal with y = 0.619. This indicates that a significant amplification is occurring.

B. The above experiment was repeated except that 100 nmoles of each dNTP was added to a 100 µl reaction, no radiolabel was employed, and aliquots were not removed at each cycle. After 10 cycles the reaction was terminated by boiling for two minutes and rehybridization was performed at 57 ° C for one hour. The sequence of the 110-bp product was confirmed by subjecting 8 µl aliquots to restriction analysis by addition of 1 µl bovine serum albumin (25 mg/ml) and 1 µl of the appropriate restriction enzyme (HinfI, MnlI, MstII, NcoI) and by reaction at 37 ° C for 15 hours. PAGE was performed as described above.

EXAMPLE 8

This example illustrates the use of different primers to amplify various fragments of pBR328 and 322.

A. The experiment described in Example 7A was repeated except using the following primers: d-(TTTGCTTCTGACACAACTGTGTTCACTAGC) and d(GCCTCACCACCAACTTCATCCACGTTCACC) to produce a 130-bp fragment of pBR328.

B. The experiment described in Example 7A was repeated except using the following primers: d-(GGTTGGCCAATCTACTCCCAGG) and d(TGGTCTCCTTAAACCTGTCTTG) to produce a 262-bp fragment of pBR328. The reaction time was 20 minutes per cycle.

C. The experiment described in Example 8B was repeated except that 100 fmoles of an MstII digest of pBR328 containing a 1.9 kb insert from the human beta-globin S allele was used as initial template. This plasmid was cleaved several times by MstII but not inside the sequence to be amplified. In addition, the primers employed were as follows:

d(GGTTGGCCAATCTACTCCCAGG) and
d(TAACCTTGATACCAACCTGCCC)

to produce a 240-bp fragment.

D. The experiment described in Example 7B was repeated except that 100 fmoles of an NruI digest of pBR322 was used as template, 200 nmoles of each dNTP were used in the 100 µl reaction, and the primers were:

d(TAGGCGTATCACGAGGCCCT) and
d(CTTCCCCATCGGTGATGTCG)

to produce a 500-bp fragment from pBR322. Reaction times were 20 minutes per cycle at 37°C. Final rehybridization was 15 hours at 57°C. Electrophoresis was on a 4% agarose gel.

EXAMPLE 9

This example illustrates the invention process wherein an in vitro mutation is introduced into the amplified segment.

A. A total of 100 fmoles of pBR322 linearized with NruI, 1 nmole each of the primers:

d(CGCATTAAAGCTTATCGATG) and
d(TAGGCGTATCACGAGGCCCT)

designed to produce a 75-bp fragment, 100 nmole each dNTP, in 100 µl 40 mM Tris at pH 8, 20 mM in $MgCl_2$, 5 mM in dithiothreitol, and 5 mg/ml bovine serum albumin were combined. The mixture was brought to 100°C for one minute, cooled for 0.5 minutes in a water bath at 23°C, whereupon 4.5 units Klenow fragment and 0.09 units inorganic pyrophosphatase were added, and a reaction was allowed to proceed for three minutes. The cycle of heating, cooling, adding enzymes, and reacting was repeated nine times. The tenth reaction cycle was terminated by freezing and an 8-µl aliquot of the reaction mixture was applied to a 4% agarose gel visualized with ethidium bromide.

B. The experiment described in Example 9A was repeated except that the oligonucleotide primers employed were:

d(CGCATTAAAGCTTATCGATG) and

d(AATTAATACGACTCACTATAGGGAGATAGGCGTATCACGAGGCCCT).

These primers are designed to produce a 101-bp fragment, 26 nucleotides of which (in the second listed primer) are not present in pBR322. These nucleotides represent the sequence of the T7 promoter, which was appended to the 75-bp sequence from pBR322 by using the primer with 20 complementary bases

and a 26-base 5' extension. The procedure required less than two hours and produced two picomoles of relatively pure 101-bp fragment from 100 fmoles of pBR322.

The T7 promoter can be used to initiate RNA transcription. T7 polymerase may be added to the 101-bp fragment to produce single-stranded RNA.

C. The experiment described in Example 8D was repeated except that the oligonucleotide primers employed were as follows:

d(TAGGCGTATCACGAGGCCCT) and

d(CCAGCAAGACGTAGCCCAGC)

to produce a 1000-bp fragment from pBR322.

D. The experiment described in Example 9C was repeated except that the oligonucleotide primers employed were as follows:

d(TAGGCGTATCACGAGGCCCT) and

d(AATTAATACGACTCACTATAGGGAGATAGGCGTATCACGAGGCCCT)

so as to produce a 1026-bp fragment, 26 nucleotides of which (in the second listed primer) are not present in pBR322 and represent the T7 promoter described above. The promoter has been inserted adjacent to a 1000-bp fragment from pBR322.

The results indicate that a primer which is not a perfect match to the template sequence but which is nonetheless able to hybridize sufficiently to be enzymatically extended produces a long product which contains the sequence of the primer rather than the corresponding sequence of the original template. The long product serves as a template for the second primer to introduce an in vitro mutation. In further cycles this mutation is amplified with an undiminished efficiency, because no further mispaired primings are required. In this case, a primer which carries a non-complementary extension on its 5' end was used to insert a new sequence in the product adjacent to the template sequence being copied.

EXAMPLE 10

This example illustrates employing nested sets of primers to decrease the background in the amplification of single copy genes.

Whole human DNA homozygous for the wild-type β-globin allele was subjected to twenty cycles of amplification as follows: a total of 10 μg DNA, 200 picomoles each of the primers:

d(ACACAACTGTGTTCACTAGC) and
d(CAACTTCATCCACGTTCACC)

and 100 nanomoles each dNTP in 100 μl of 30 mM Tris-acetate pH 7.9, 60 mM sodium acetate, 10 mM dithiothreitol, and 10 mM magnesium acetate were heated to 100 °C for one minute, cooled to 25 °C for one minute, and treated with 2 units Klenow fragment for two minutes. The cycle of heating, cooling and adding Klenow was repeated 19 times. A ten-μl aliquot was removed from the reaction mixture and subjected to a further ten cycles of amplification using each of the primers:

d(CAGACACCATGGTGCACCTGACTCCTG) and
d(CCCCACAGGGCAGTAACGGCAGACTTCTCC),

which amplify a 58-bp fragment contained within the 110-bp fragment produced above. This final ten cycles of amplification was accomplished by diluting the 10-μl aliquot into 90 μl of the fresh Tris-acetate buffer

described above containing 100 nanomoles each dNTP and 200 pmoles of each primer. Reaction conditions were as above. After ten cycles a 10-μl aliquot (corresponding to 100 nanograms of the original DNA) was applied to a 6% NuSieve (FMC Corp.) agarose gel and visualized using ethidium bromide.

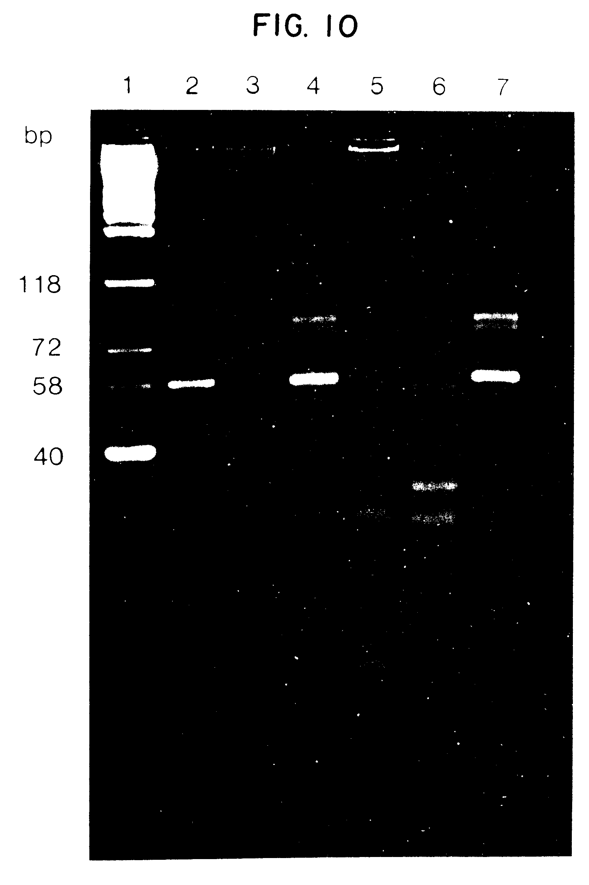

Figure 10 illustrates this gel illuminated with UV light and photographed through a red filter as is known in the art. Lane 1 is molecular weight markers. Lane 2 is an aliquot of the reaction described above. Lane 3 is an aliquot of a reaction identical to that described above, except that the original wild-type DNA was cleaved with DdeI prior to amplification. Lane 4 is an aliquot of a reaction identical to that described above, except that human DNA homozygous for the sickle betaglobin allele was treated with DdeI prior to amplification (the sickle allele does not contain a DdeI site in the fragment being amplified here). Lane 5 is an aliquot of a reaction identical to that described above, except that salmon sperm DNA was substituted for human DNA. Lane 6 is an aliquot of a reaction identical to that described above, except that the aliquot was treated with DdeI after amplification (DdeI should convert the 58-bp wild-type product into 27-and 31-bp fragments). Lane 7 is an aliquot of the Lane 4 material treated with DdeI after amplification (the 58-bp sickle product contains no DdeI site).

Detection of a 58-bp fragment representative of a single-copy gene from one microgram of human DNA using only ethidium bromide staining of an agarose gel requires an amplification of about 500,000-fold. This was accomplished by using the two nested sets of oligonucleotide primers herein. The first set amplifies the 110-bp fragment and the inner nested set amplifies a sub-fragment of this product up to the level of convenient detection shown in Figure 10. This procedure of using primers amplifying a smaller sequence contained within the sequence being amplified in the previous amplification process and contained in the extension products of the other primers allows one to distinguish the wild-type from the sickle allele at the beta-globin locus without resorting to either radioisotopic or non-radioisotopic probe hybridization methodology such as that of Conner et al., PNAS (USA), 80:278 (1983) and Leary et al., PNAS (USA), 80:4045 (1983).

## EXAMPLE 11

The present process is expected to be useful in detecting, in a patient DNA sample, a specific sequence associated with an infectious disease such as, e.g., Chlamydia using a biotinylated hybridization probe spanning the desired amplified sequence and using the process described in U.S. 4,358,535, supra. The biotinylated hybridization probe may be prepared by intercalation and irradiation of a partially double-stranded DNA with a 4'-methylene substituted 4,5'-8-trimethylpsoralen attached to biotin via a spacer arm of the formula:

$$-Y-(CH_2)_2-O-[CH_2)_xO]_y-CH_2CH_2-\overset{H}{\overset{|}{N}}-$$

where Y is O, NH or N-CHO, x is a number from 1 to 4, and y is a number from 2 to 4. Detection of the biotinyl groups on the probe may be accomplished using a streptavidin-acid phosphatase complex commercially obtainable from Enzo Biochem Inc. using the detection procedures suggested by the manufacturer in its brochure. The hybridized probe is seen as a spot of precipitated stain due to the binding of the detection complex, and the subsequent reaction catalyzed by acid phosphatase, which produces a precipitable dye.

## EXAMPLE 12

In this example, the process of Example 7 was basically used to amplify a 119 base pair fragment on the human β-hemoglobin gene using the primers:

5'-CTTCTGcagCAACTGTGTTCACTAGC-3' (GH18)
5'-CACaAgCTTCATCCACGTTCACC-3' (GH19)

where lower case letters denote mismatches from wild-type sequence to create restriction enzyme sites. The full scheme is shown in Table I. Table I illustrates a diagram of the primers GH18 and GH19 which are used for cloning and sequencing a 119-base pair fragment of the human $\beta$-globin gene and which are designed to contain internal restriction sites. The start codon ATG is underlined. GH18 is a 26-base oligonucleotide complementary to the negative strand and contains an internal PstI site. GH19 is a 23-base oligonucleotide complementary to the plus strand and contains an internal HindIII recognition sequence. Arrows indicate the direction of extension by DNA polymerase I. The boxed sequences indicate the restriction enzyme recognition sequences of each primer. These primers were selected by first screening the regions of the gene for homology to the PstI and HindIII restriction sites of bacteriophage M13. The primers were then prepared as described in previous examples.

TABLE I

GH19

DdeI

←———— CCACTTGCACCTAcTTCgAaCAC

CTTCTGACACAACTGTGTTCACTAGCAACCTCAAACAGACACCATGGTGCACCTGACTCCTGAGGAGAAGTCTGCCGTTACTGCCCTGTGGGGCAAGGTGAACGTGGATGAAGTTGGTG(+)

GAAGACTGTGTTGACACAAGTGATCGTTGGAGTTTGTCTGTGGTACCACGTGGACTGAGGACTCCTCTTCAGACGGCAATGACGGGACACCCCGTTCCACTTGCACCTACTTCAACCAC(-)

CTTCTGcagCAACTGTGTTCACTAGC ————→

GH18

Pst I

5' CTTCTGcagCAACTGTGTTCACTAGC 3' GH18 left linker primer

5' CACaAgCTTcATCCACGTTCACC 3' GH19 right linker primer

Hind III

Amplification and Cloning

After twenty cycles of amplification of 1 microgram of human genomic DNA isolated from the cell line Molt 4 as described in Example 2, 1/14th of the reaction product was hybridized to the labeled β-globin specific oligonucleotide probe, RS06, of the sequence 5'-CTGACTCCTGAGGAG AAGTCTGCCGTTACTGCCCTGTGGG-3' using the methods described above for oligomer restriction. Following solution hybridization, the reaction mixture was treated with DdeI under restriction digestion conditions as described above, to produce an 8-base pair oligonucleotide. The amount of this 8-base pair product is proportional to the amount of amplified product produced. The digestion products were resolved on a 30% polyacrylamide gel and visualized by autoradiography.

Analysis of the autoradiogram revealed that the amplification was comparable in efficiency to that of amplification with primers PC03 (5'-ACACAACTGTGTTCACTAGC-3') and PC04 (5'-CCACTTGCACCTACTTCAAC-3'), which are complementary to the negative and positive strands, respectively, of the wild-type β-globin.

The amplified product was ethanol precipitated to desalt and concentrate the sample, redissolved in a restriction buffer of 10 mM Tris pH 8, 10 mM $MgCl_2$, 1 mM DTT, 100 mM $NaCl_2$, and simultaneously digested with PstI and HindIII. After digestion the sample was desalted with a Centricon 10 concentrator and ligated overnight at 12°C with 0.3 micrograms of the PstI/HindIII digested vector M13mp10w, which is publicly available from Boehringer-Mannheim.

The entire ligation mixture was transformed into E. coli strain JM103, which is publicly available from BRL in Bethesda, MD. The procedure followed for preparing the transformed strain is described in Messing, J. (1981) Third Cleveland Symposium on Macro-molecules:Recombinant DNA, ed. A. Walton, Elsevier, Amsterdam, 143-153.

The transformation mixture was plated onto x-gal media for screening via plaque hybridization with nylon filters. The filters were probed with a β-globin-specific oligonucleotide probe RS24 of the sequence 5'-CCCACAGGGCAGTAACGGCAGACTTCTCCTCAGGAGTCAG-3' to determine the number of β-globin inserts. The filters were then reprobed with the primer PC04 to determine the total number of inserts.

Plating and Screening

Table II summarizes the plating and plaque hybridization data. The filters were probed with the primer PC04 to determine the percentage of inserts resulting from amplification and cloning: 1206 clear plaques (90% of total number of clear plaques) hybridized to the primer. Fifteen plaques hybridized to the β-globin specific probe RS24. The percentage of β-globin positive plaques among the amplified primer-positive plaques is approximately 1%.

TABLE II

| Plate No. | Blue Plaques | No Inserts* | Inserts** | β-Globin Inserts |
|---|---|---|---|---|
| 1 | 28 | 25 | 246 | 1 |
| 2 | 29 | 18 | 222 | 2 |
| 3 | 11 | 26 | 180 | 0 |
| 4 | 24 | 20 | 192 | 5 |
| 5 | 22 | 27 | 185 | 5 |
| 6 | 39 | 21 | 181 | 3 |
| TOTAL | 158 | 132 | 1206 | 15 |

% of plaques containing amplified sequences which contain β-globin insert = 15/1206 x 100 = 1.24%

% of total plaques which contain β-globin insert = 15/1496 x 100 = ca.1%

% of total plaques which contain amplified sequences = 1206/1496 x 100 = 0.8%

* Clear plaques which do not hybridize to primer PC04

** Clear plaques which hybridize to primer PC04

Restriction Enzyme and Southern Blot Analysis

DNA from phage DNA minipreparation of three β-globin positive and two β-globin negative (but PC04 primer positive) plaques were analyzed by restriction enzyme analysis. MstII digestion of DNA from M13 clones containing the amplified β-globin fragment should generate a characteristic 283 base-pair fragment. Following MstII digestion, the three β-globin positive clones all produced the predicted 283 base pair fragment, while the two clones which were positive only with the primer produced larger fragments.

The gel from this analysis was transferred to a MSI nylon filter and hybridized with a radiolabeled nick-translated β-globin probe prepared by standard nick translation methods as described by Rigby et al., J. Mol. Biol. (1977), 113:237-51. The only bands which hybridized to the β-globin probe were the three β-globin positive clones. The two other clones had inserts which did not hybridize to the β-globin probe.

Sequence Analysis

Ten β-globin positive clones which were shown by restriction enzyme analysis to contain the β-globin insert were sequenced using the M13-dideoxy sequencing method. Of the ten clones, nine were identical to the β-globin wild-type sequence. The other clone was identical to the δ-globin gene which had been shown to be amplified to only a small degree by the β-globin primers.

In conclusion, the modified linker primers were nearly as efficient as the unmodified primers in amplifying the β-globin sequence. The primers were able to facilitate insertion of amplified DNA into cloning vectors. Due to the amplification of other segments of the genome, only 1% of the clones contained hemoglobin sequences.

Nine of the ten clones were found to be identical to the published β-globin sequence, showing that the technique amplifies genomic DNA with high fidelity. One clone was found to be identical with the published δ-globin sequence, confirming that the primers are specific for the β-globin gene despite their having significant sequence homology with δ-globin.

When cloning was carried out with a 267 base pair fragment of the β-globin gene, cloning was effective only when dimethylsulfoxide was present (10% by volume at 37°C) in the amplification procedure.

Restriction site-modified primers were also used to amplify and clone and partially sequence the human N-ras oncogene and to clone 240-base pair segments of the HLA DQ-α and DQ-β genes. All of these amplifications were carried out in the presence of 10% by volume dimethylsulfoxide at 37°C. The primers for amplifying HLA DQ-α and DQ-β genes were much more specific for their intended targets than were the β-globin and DR-β primers, which, rather than giving a discrete band on an ethidium bromide stained

agarose gel, produced only a smear. In addition, the HLA DQ-α primers produced up to 20% of clones, with amplified inserts which contained the desired HLA target fragment, whereas 1% of the β-globin clones contained the target sequence. The HLA DQ-α and DQ-β gene cloning was only effective when the DMSO was present and the temperature was elevated.

EXAMPLE 13

This example illustrates the use of the process herein to prepare the TNF gene of 494 base pairs starting from two oligonucleotides of 74 base pairs each.

PRIMERS

The primers employed were prepared by the method described in Example 2 and are identified below, each being 74 mers.

(TN10) 5'-CCTCGTCTACTCCCAGGTCCTCTTCAAGGGCCAAGGCTGCCCCGACTATGTGCTCCTCA-CCCACACCGTCAGCC-3'

(TN11) 5'-GGCAGGGGCTCTTGACGGCAGAGAGGAGGTTGACCTTCTCCTGGTAGGAGATGGCGAAG-CGGCTGACGGTGTGG-3'

(LL09) 5'-CCTGGCCAATGGCATGGATCTGAAAGATAACCAGCTGGTGGTGCCAGCAGATGGCCTGT-ACCTCGTCTACTCCC-3'

(LL12) 5'-CTCCCTGATAGATGGGCTCATACCAGGGCTTGAGCTCAGCCCCCTCTGGGGTGTCCTTC-GGGCAGGGGCTCTTG-3'

(TN08) 5'-TGTAGCAAACCATCAAGTTGAGGAGCAGCTCGAGTGGCTGAGCCAGCGGGCCAATGCCC-TCCTGGCCAATGGCA-3'

(TN13) 5'-GATACTTGGGCAGATTGACCTCAGCGCTGAGTTGGTCACCCTTCTCCAGCTGGAAGACC-CCTCCCTGATAGATG-3'

(LL07) 5'-CCTTAAGCTTATGCTCAGATCATCTTCTCAAAACTCGAGTGACAAGCCTGTAGCCCATG-TTGTAGCAAACCATC-3'

(TN14) 5'-GCTCGGATCCTTACAGGGCAATGACTCCAAAGTAGACCTGCCCAGACTCGGCAAAGTCG-AGATACTTGGGCAGA-3'

OVERALL PROCEDURE

I. Ten cycles of the protocol indicated below were carried out using primers TN10 and TN11, which interact as shown in the diagram below, step (a).

II. A total of 2 µl of the reaction mixture from Part I above was added to the primers LL09 and LL12. The protocol described below was carried out for 15 cycles, so that the primers would interact with the product of Part I as shown in the diagram below, step (b).

III. A total of 2 µl of the reaction mixture from Part II above was added to the primers TN08 and TN13. The protocol described below was carried out for 15 cycles, so that the primers would interact with the product of Part II as shown in the diagram below, step (c).

IV. A total of 2 µl of the reaction mixture from Part III above was added to the primers LL07 and LL14. The protocol described below was carried out for 15 cycles, so that the primers would interact with the product of Part III as shown in the diagram below, step (d).

PROTOCOL

Each reaction contained 100 µl of:
2 mM of each of dATP, dCTP, dGTP and dTTP
3 µM of each of the primers used at that step
1 x polymerase buffer, (30 mM Tris-acetate, 60 mM Na-acetate, 10 mM Mg-acetate, 2.5 mM dithiothreitol)

Each cycle constituted:

1) 1 min. in boiling water
2) 1 min. cooling at room temperature
3) add 1 µl (5 units) of the Klenow fragment of DNA polymerase
4) allow the polymerization reaction to proceed for 2 min. For the next cycle start again at step 1.

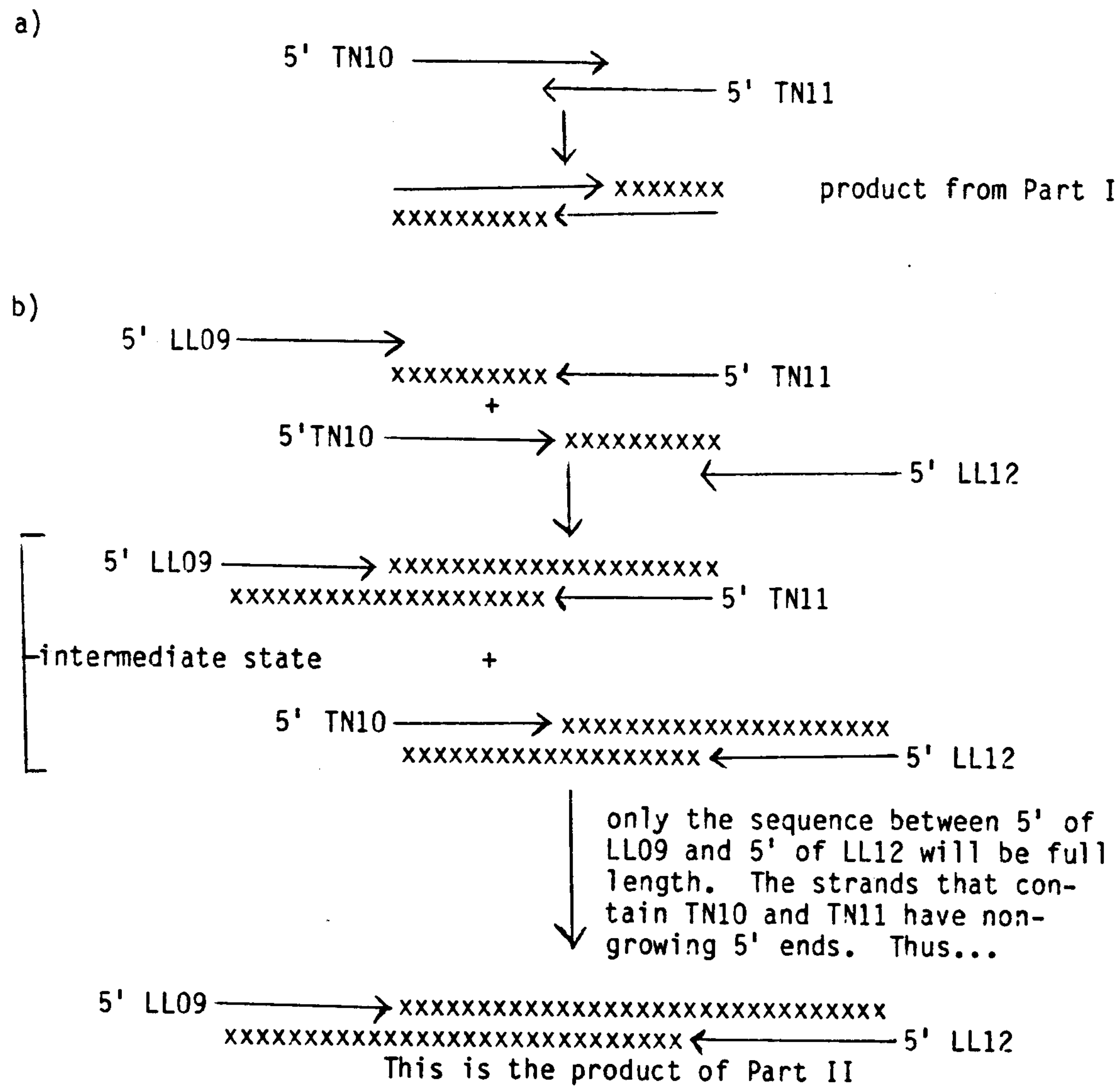


DIAGRAM

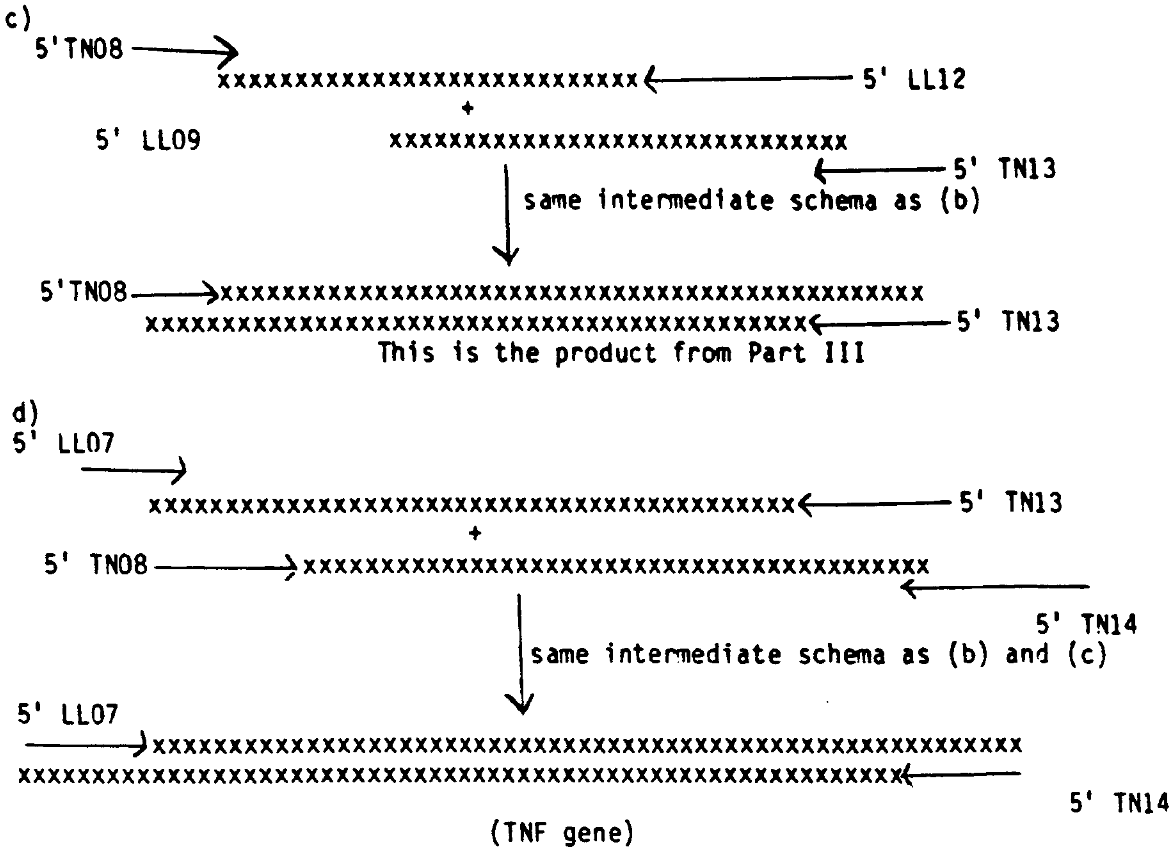


In summary, the present invention is seen to provide a process for detecting sequences in nucleic acids by first amplifying one or more specific nucleic acid sequences using a chain reaction in which primer extension products are produced which can subsequently act as templates for further primer extension reactions. The process is especially useful in detecting nucleic acid sequences which are initially present in only very small amounts. Also, the amplification process can be used for molecular cloning.

## Claims

**1.** A process for detecting the presence or absence of at least one specific double-stranded nucleic acid sequence in a sample, or distinguishing between two different double-stranded nucleic acid sequences in said sample, which process comprises first exponentially amplifying the specific sequence or sequences (if present) by the following steps, and then detecting the thus-amplified sequence or sequences (if present):

(a) separating the nucleic acid strands in the sample and treating the sample with a molar excess of a pair of oligonucleotide primers for each different specific sequence being detected, one primer for each strand, under hybridizing conditions and in the presence of an inducing agent for polymerization and the different nucleoside triphosphates such that for each of said strands an extension product of the respective primer is synthesized which is complementary to the strand, wherein said primers are selected so that each is substantially complementary to one end of the sequence to be amplified on one of the strands such that the extension product synthesized from one primer, when it is separated from its complement, can serve as a template for synthesis of an extension product of the other primer of the pair;

(b) treating the sample resulting from (a) under denaturing conditions to separate the primer extension products from their templates;

(c) treating as in (a) the sample resulting from (b) with oligonucleotide primers such that a primer extension product is synthesized using each of the single strands produced in step (b) as a template; and, if desired,

(d) repeating steps (b) and (c) at least once;

whereby exponential amplification of the nucleic acid sequence or sequences, if present, results thus

permitting detection thereof; and, if desired,
(e) adding to the product of step (c) or (d) a labelled oligonucleotide probe capable of hybridizing to said sequence to be detected; and
(f) determining whether said hybridization has occurred.

**2.** A process of Claim 1, wherein a sequence to be amplified and detected is contained within a larger sequence.

**3.** A process of Claim 1 or Claim 2, wherein the agent for polymerization is E. coli DNA polymerase I, Klenow fragment of E. coli DNA polymerase I, T4 DNA polymerase, reverse transcriptase, or a heat-stable enzyme.

**4.** A process of any one of Claims 1-3, wherein the nucleic acid is DNA and the primers are oligodeoxyribonucleotides.

**5.** A process of any one of Claims 1-4, wherein each primer employed contains a restriction site which is the same as or different from a restriction site the other primer, and after step (c) or (d) and before step (e) the product of step (c) or (d) (as the case may be) is cleaved with a restriction enzyme specific for each of said restriction sites and the cleaved products are separated from the uncleaved products and used in step (e).

**6.** A process of any one of Claims 1-5, wherein a sequence to be amplified and detected is associated with a genetic, cancerous or infectious disease.

**7.** A process of any one of Claims 1-6, wherein a single-stranded nucleic acid sequence which it is desired to detect is first treated under hybridizing conditions with a primer, inducing agent for polymerization and the different nucleoside triphosphates, to form a complementary strand thereto thereby to provide said starting double-stranded nucleic acid sequence and to permit detection of said single-stranded sequence.

**8.** A process for cloning into a vector a specific double-stranded nucleic acid sequence or sequences contained in a nucleic acid or a mixture of nucleic acids, which process comprises first amplifying said sequence or sequences, by the following steps:
(a) separating the nucleic acid strands and treating the nucleic acid(s) with a molar excess of a pair of oligonucleotide primers for each different specific sequence being amplified and cloned, one for each strand, under hybridizing conditions and in the presence of an inducing agent for polymerization and the different nucleoside triphosphates such that for each of said strands an extension product of each primer is synthesized which is complementary to its respective strand, wherein said primers are selected so that each is substantially complementary to one end of the sequence to be amplified on one of the strands such that the extension product synthesized from one primer, when it is separated from its complement, can serve as a template for synthesis of the extension product of the other primer of the pair and wherein said primers each contain a restriction site which is the same as or different from the restriction site(s) on the other primer(s);
(b) separating the primer extension products from the templates on which they were synthesized to produce single-stranded molecules;
(c) treating as in (a) the single-stranded molecules generated from step (b) with oligonucleotide primers such that a primer extension product is synthesized using each of the single strands produced in step (b) as a template; and, if desired,
(d) repeating steps (b) and (c) at least once;
whereby exponential amplification of the nucleic acid sequence or sequences results;
(e) adding to the product of step (c) or (d) a restriction enzyme for each of said restriction sites to obtain cleaved products in a restriction digest; and
(f) ligating the cleaved product(s) into one or more cloning vectors.

**9.** A process for synthesizing a nucleic acid fragment from an existing double-stranded nucleic acid sequence having fewer nucleotides than the fragment being synthesized and two oligonucleotide primers, wherein the nucleic acid being synthesized is comprised of a left segment, a core segment and a right segment, and wherein the core segment represents at least substantially the nucleotide

sequence of said existing nucleic acid sequence, and the right and left segments represent the nucleotide sequence present at one end of each of the two primers, the other ends of which are complementary or substantially complementary to ends of the single strands produced by separating the strands of said existing nucleic acid sequence, which process comprises:

(a) treating the strands of said existing fragment with a molar excess of two oligonucleotide primers, one for each of the strands, under hybridizing conditions and in the presence of an inducing agent for polymerization and the different nucleoside triphosphates such that for each strand an extension product of the respective primer is synthesized which is complementary to the strand, wherein said primers are selected so as each to be substantially complementary to one end of a strand of said existing sequence such that the extension product synthesized from one primer, when it is separated from its complement, can serve as a template for synthesis of an extension product of the other primer, and wherein each primer also contains a sequence of nucleotides which are not complementary to said existing sequence and which correspond to the two ends of the nucleic acid fragment being synthesized;

(b) separating the primer extension products from the templates on which they were synthesized to produce single-stranded molecules; and

(c) treating the single-stranded molecules generated from step (b) with the primers of step (a) under conditions such that a primer extension product is synthesized using each of the single strands produced in step (b) as a template so as to produce two intermediate double-stranded nucleic acid molecules, into each of which has been incorporated the nucleotide sequence present at one end of the oligonucleotide primers, and two full-length double-stranded nucleic acid molecules, into each of which has been incorporated the nucleotide sequence present at one end of both of the oligonucleotide primers;

(d) repeating steps (b) and (c) for a sufficient number of times to produce the full-length double-stranded molecules in an effective amount.

**10.** A process as claimed in Claim 9, wherein the product of step (d) is then used to produce a yet further extended nucleic acid sequence by extending it in accordance with a process as defined in Claim 9.

**11.** A process as claimed in Claim 9 or Claim 10, wherein the existing nucleic acid sequence is obtained by treating two oligonucleotides, each of which has at one end thereof a nucleotide sequence which is complementary to an end of the other oligonucleotide and which are non-complementary to each other at their other ends, with an agent for polymerization and the different nucleoside triphosphates under conditions such that an extension product of each oligonucleotide is synthesized which is complementary to the other of the resulting extension products.

**12.** A process according to any one of Claims 8 to 10, wherein a single-stranded nucleic acid sequence is first treated under hybridizing conditions with a primer, inducing agent for polymerization and the different nucleoside triphosphate, to form a complementary strand thereto thereby to provide said starting double-stranded nucleic acid sequence.

**13.** A kit for the detection of at least one specific nucleic acid sequence in a sample, which kit comprises, in packaged form, a multicontainer unit having:

(a) each of two oligonucleotide primers for each different sequence to be detected, wherein

(i) if the specific nucleic acid sequence to be detected is single-stranded one primer is substantially complementary to one end of the strand so that an extension product of said one primer formed under hybridizing conditions and in the presence of an inducing agent for polymerization and the different nucleoside triphosphates is substantially complementary to said strand, and the other primer is substantially complementary to one end of said extension product and can be used under hybridizing conditions and in the presence of an inducing agent for polymerization and the different nucleoside triphosphates to synthesize another extension product employing said extension product of said one primer as a template thereby providing a nucleic acid consisting of two strands; or

(ii) if the specific nucleic acid sequence to be detected is double-stranded the primers are such that each is substantially complementary to one end of one of the strands and an extension product synthesized from each primer using its complement strand as a template under hybridizing conditions and in the presence of an inducing agent for polymerization and the different nucleoside triphosphates, when separated from its complement, can serve as a template

for synthesis of an extension product of the other primer under hybridizing conditions and in the presence of an inducing agent for polymerization and the different nucleoside triphosphates;
(b) an agent for polymerization;
(c) each of the different nucleoside triphosphates;
(d) an oligonucleotide probe capable of hybridizing to said sequence if it is present in said sample; and
(e) means for detecting hybrids of said probe and said sequence.

## Patentansprüche

1. Verfahren zum Nachweis der An- oder Abwesenheit von mindestens einer spezifischen doppelsträngigen Nucleinsäuresequenz in einer Probe oder zur Unterscheidung zwischen zwei verschiedenen doppelsträngigen Nucleinsäuresequenzen in der Probe, wobei das Verfahren zuerst ein exponentielles Amplifizieren der spezifischen Sequenz oder Sequenzen (wenn vorhanden) durch die nachfolgenden Schritte und dann ein Nachweisen der so amplifizierten Sequenz oder Sequenzen (wenn vorhanden) umfaßt:
(a) Trennen der Nucleinsäurestränge in der Probe und Behandeln der Probe mit einem molaren Überschuß eines Paares von Oligonucleotid-Primern für jede unterschiedliche nachzuweisende spezifische Sequenz, mit einem Primer für jeden Strang, unter Hybridisierungsbedingungen und in Gegenwart eines induzierenden Agens für die Polymerisation und der verschiedenen Nucleosidtriphosphate, so daß von dem betreffenden Primer für jeden der Stränge ein zum Strang komplementäres Verlängerungsprodukt synthetisiert wird, wobei die Primer so ausgewählt werden, daß jeder im wesentlichen komplementär zu einem Ende der zu amplifizierenden Sequenz auf einem der Stränge ist, damit das von einem Primer synthetisierte Verlängerungsprodukt, wenn es von seinem Komplement abgetrennt wird, als Matrize zur Synthese eines Verlängerungsproduktes des anderen Primers des Paares dienen kann;
(b) Behandeln der aus (a) hervorgehenden Probe unter Denaturierungsbedingungen, um die Primer-Verlängerungsprodukte von ihren Matrizen zu trennen;
(c) Behandeln der aus (b) hervorgehenden Probe, wie in (a) mit Oligonucleotid-Primern, so daß unter Verwendung aller im Schritt (b) produzierten Einzelstränge als Matrize ein Primer-Verlängerungsprodukt synthetisiert wird; und, falls gewünscht,
(d) ein mindestens einmaliges Wiederholen der Schritte (b) und (c), wodurch sich eine exponentielle Amplifikation der Nucleinsäuresequenz oder -sequenzen (wenn vorhanden) ergibt, und somit deren Nachweis erlaubt; und, falls gewünscht,
(e) Zugeben einer markierten Oligonucleotid-Sonde zum Produkt von Schritt (c) oder (d), die fähig ist, mit der nachzuweisenden Sequenz zu hybridisieren; und
(f) Bestimmen, ob die Hybridisierung stattgefunden hat.

2. Verfahren nach Anspruch 1, wobei eine zu amplifizierende und nachzuweisende Sequenz innerhalb einer größeren Sequenz enthalten ist.

3. Verfahren nach einem der Ansprüche 1 oder 2, wobei das Agens für die Polymerisation E. coli-DNA-Polymerase I, Klenow-Fragment von E. coli-DNA-Polymerase I, T4-DNA-Polymerase, Reverse Transkriptase oder ein hitzestabiles Enzym ist.

4. Verfahren nach einem der Ansprüche 1 bis 3, wobei die Nucleinsäure DNA ist, und die Primer Oligodesoxyribonucleotide sind.

5. Verfahren nach einem der Ansprüche 1 bis 4, wobei jeder der verwendeten Primer eine Restriktionsstelle enthält, die mit der Restriktionsstelle des anderen Primers gleich oder von ihr verschieden ist und das Produkt von Schritt (c) oder (gegebenenfalls) (d) nach Schritt (c) oder (d) und vor Schritt (e) mit einem für jede der Restriktionsstellen spezifischen Restriktionsenzym gespalten wird, und die gespaltenen Produkte von den ungespaltenen Produkten abgetrennt und in Schritt (e) verwendet werden.

6. Verfahren nach einem der Ansprüche 1 bis 5, wobei eine zu amplifizierende und nachzuweisende Sequenz mit einer genetischen, einer Krebs- oder Infektionskrankheit assoziiert ist.

7. Verfahren nach einem der Ansprüche 1 bis 6, wobei eine einzelsträngige Nucleinsäuresequenz, die

man nachweisen möchte, zunächst unter Hybridisierungsbedingungen mit einem Primer, einem induzierenden Agens für die Polymerisation und den verschiedenen Nucleosidtriphosphaten behandelt wird, um einen dazu komplementären Strang zu erzeugen, um so die doppelsträngige Ausgangs-Nucleinsäuresequenz bereitzustellen und den Nachweis der einzelsträngigen Sequenz zu erlauben.

**8.** Verfahren zur Clonierung in einem Vektor einer spezifischen doppelsträngigen Nucleinsäuresequenz oder -sequenzen, die in einer Nucleinsäure oder in einem Gemisch von Nucleinsäuren enthalten ist/sind, wobei das Verfahren zuerst das Amplifizieren der Sequenz oder Sequenzen durch die nachfolgenden Schritte umfaßt:

(a) Trennen der Nucleinsäurestränge und Behandeln der Nucleinsäure(n) mit einem molaren Überschuß eines Paares von Oligonucleotid-Primern für jede zu amplifizierende und zu clonierende unterschiedliche spezifische Sequenz, jeweils einer für jeden Strang, unter Hybridisierungsbedingungen und in Gegenwart eines induzierenden Agens für die Polymerisation und der verschiedenen Nucleosidtriphosphate, so daß für jeden der Stränge ein Verlängerungsprodukt von jedem Primer synthetisiert wird, das zu seinem betreffenden Strang komplementär ist, wobei die genannten Primer so ausgewählt sind, daß jeder im wesentlichen zu einem Ende der zu amplifizierenden Sequenz auf einem der Stränge komplementär ist, so daß das von einem Primer synthetisierte Verlängerungsprodukt, wenn es von seinem Komplement getrennt ist, als eine Matrize zur Synthese des Verlängerungsproduktes des anderen Primers des Paares dienen kann, und wobei jeder der Primer eine Restriktionsstelle enthält, die gleich oder verschieden von der Restriktionsstelle (den Restriktionsstellen) auf dem anderen Primer (den anderen Primern) ist;

(b) Trennen der Primer-Verlängerungsprodukte von den Matrizen, an denen sie synthetisiert worden sind, um einzelsträngige Moleküle zu produzieren;

(c) Behandeln der einzelsträngigen in Schritt (b) erzeugten Moleküle wie in (a) mit Oligonucleotid-Primern, so daß unter Verwendung aller in Schritt (b) produzierten Einzelstränge als Matrize ein Primer-Verlängerungsprodukt synthetisiert wird; und, falls gewünscht,

(d) ein mindestens einmaliges Wiederholen der Schritte (b) und (c) wodurch sich eine exponentielle Amplifikation der Nucleinsäuresequenz oder -sequenzen ergibt;

(e) Zugeben eines Restriktionsenzyms für jede der genannten Restriktionsstellen zum Produkt von Schritt

(c) oder (d), um in einem Restriktionsverdaugespaltene Produkte zu erhalten; und

(f) Ligieren des (der) gespaltenen Produktes (Produkte) mit einem oder mehreren Clonierungsvektoren.

**9.** Verfahren zur Synthese eines Nucleinsäurefragments aus einer vorhandenen doppelsträngigen weniger Nucleotide als das in der Synthese befindliche Fragment aufweisenden Nucleinsäuresequenz und zwei Oligonucleotid-Primern, wobei die in der Synthese befindliche Nucleinsäure einen linken Abschnitt, einen Kernabschnitt und einen rechten Abschnitt enthält und wobei der Kernabschnitt mindestens im wesentlichen die Nucleotidsequenz der vorhandenen Nucleinsäuresequenz darstellt und der rechte und der linke Abschnitt die Nucleotidsequenz darstellt, die an einem Ende von jedem der beiden Primer vorhanden ist, deren andere Enden komplementär oder im wesentlichen komplementär zu den Enden der durch Strangtrennung der bestehenden Nucleinsäuresequenz produzierten Einzelstränge ist, wobei das Verfahren die nachfolgenden Schritte umfaßt:

(a) Behandeln der Stränge des vorhandenen Fragments mit einem molaren Überschuß zweier Oligonucleotid-Primer - einer für jeden der Stränge - unter Hybridisierungsbedingungen und in Gegenwart eines induzierenden Agens für die Polymerisation und der verschiedenen Nucleosidtriphosphate, so daß für jeden Strang von dem betreffenden Primer ein zu dem Strang komplementäres Verlängerungsprodukt synthetisiert wird, wobei die Primer so ausgewählt sind, daß jeder im wesentlichen komplementär zu einem Ende eines Stranges der vorhandenen Sequenz ist, damit das von einem Primer synthetisierte Verlängerungsprodukt, wenn es von seinem Komplement getrennt worden ist, als Matrize zur Synthese eines Verlängerungsproduktes des anderen Primers dienen kann, und wobei jeder Primer ebenfalls eine Nucleotidsequenz enthält, die zur vorhandenen Sequenz nicht komplementär ist und mit den beiden Enden des in der Synthese befindlichen Nucleinsäurefragments korrespondiert;

(b) Trennen der Primer-Verlängerungsprodukte von den Matrizen, an denen sie synthetisiert worden sind, um einzelsträngige Moleküle zu produzieren; und

(c) Behandeln der in Schritt (b) erzeugten einzelsträngigen Moleküle mit den Primern aus Schritt (a) unter solchen Bedingungen, daß unter Verwendung einer der in Schritt (b) produzierten Einzelstränge als Matrize ein Primer-Verlängerungsprodukt synthetisiert wird, so daß zwei doppelsträngige intermediäre Nucleinsäuremoleküle, in die jeweils die an einem Ende des Oligonucleotid-Primers vorhandene Nucleinsäuresequenz eingebaut worden ist, und zwei doppelsträngige Nucleinsäuremoleküle mit voller Länge, in die jeweils die an einem Ende jedes der beiden Oligonucleotid-Primer vorhandene Nucleotidsequenz eingebaut worden ist, produziert werden;

(d) ausreichend häufiges Wiederholen der Schritte (b) und (c), um die doppelsträngigen Moleküle mit voller Länge in einer wirksamen Menge zu produzieren.

**10.** Verfahren nach Anspruch 9, wobei das Produkt von Schritt (d) dann verwendet wird, um eine noch weiter verlängerte Nucleinsäuresequenz durch dessen Verlängerung nach einem in Anspruch 9 definierten Verfahren zu produzieren.

**11.** Verfahren nach Anspruch 9 oder 10, wobei man die vorhandene Nucleinsäuresequenz erhält durch Behandeln zweier Oligonucleotide, von denen jedes an einem Ende eine zu einem Ende des anderen Oligonucleotids komplementäre Nucleotidsequenz aufweist, und die an ihren anderen Enden zueinander nicht-komplementär sind, mit einem Agens für die Polymerisation und den verschiedenen Nucleosidtriphosphaten unter solchen Bedingungen, daß von jedem Oligonucleotid ein Verlängerungsprodukt synthetisiert wird, das zu dem anderen resultierenden Verlängerungsprodukt komplementär ist.

**12.** Verfahren nach einem der Ansprüche 8 bis 10, wobei zuerst eine einzelsträngige Nucleinsäuresequenz unter Hybridisierungsbedingungen mit einem Primer, einem induzierenden Agens für die Polymerisation und den verschiedenen Nucleosidtriphosphaten behandelt wird, um so zur Bereitstellung der doppelsträngigen Ausgangs-Nucleotidsequenz einen dazu komplementären Strang zu erzeugen.

**13.** Kit zum Nachweis von mindestens einer spezifischen Nucleinsäuresequenz in einer Probe, wobei der Kit in einer gepackten Form eine Vielfachbehälter-Einheit umfaßt, aufweisend:

(a) je zwei Oligonucleotid-Primer für jede unterschiedliche nachzuweisende Sequenz, wobei

(i) falls die nachzuweisende spezifische Nucleinsäuresequenz einzelsträngig ist, ein Primer im wesentlichen komplementär zu einem Ende des Stranges ist, so daß ein unter Hybridisierungsbedingungen und in Gegenwart eines induzierenden Agens für die Polymerisation und der verschiedenen Nucleosidtriphosphate erzeugtes Verlängerungsprodukt des einen Primers im wesentlichen komplementär zu dem Strang ist, und der andere Primer im wesentlichen zu einem Ende des Verlängerungsproduktes komplementär ist und unter Hybridisierungsbedingungen und in Gegenwart eines induzierenden Agens für die Polymerisation und der verschiedenen Nucleosidtriphosphate zur Synthese eines anderen Verlängerungsproduktes verwendet werden kann, wobei das Verlängerungsprodukt des erstgenannten Primers als eine Matrize verwendet wird, wodurch eine aus zwei Strängen bestehende Nucleinsäure bereitgestellt wird; oder

(ii) falls die nachzuweisende spezifische Nucleinsäuresequenz doppelsträngig ist, die Primer so beschaffen sind, daß jeder im wesentlichen komplementär zu einem Ende eines der Stränge ist, und ein von je einem Primer unter Verwendung seines komplementären Stranges als Matrize unter Hybridisierungsbedingungen und in Gegenwart eines induzierenden Agens für die Polymerisation und der verschiedenen Nucleosidtriphosphate syntetisiertes Verlängerungsprodukt, wenn es von seinem Komplement getrennt ist, unter Hybridisierungsbedingungen und in Gegenwart eines induzierenden Agens für die Polymerisation und der verschiedenen Nucleosidtriphosphate als eine Matrize zur Synthese eines Verlängerungsproduktes des anderen Primers dienen kann;

(b) ein Agens für die Polymerisation;

(c) jedes der verschiedenen Nucleosidtriphosphate;

(d) eine Oligonucleotid-Sonde, die fähig ist, mit der genannten Sequenz, wenn sie in der Probe anwesend ist, zu hybridisieren; und

(e) Hilfsmittel für den Nachweis von Hybriden aus der Sonde und der Sequenz.

**Revendications**

**1.** Procédé pour la détection de la présence ou de l'absence d'au moins une séquence spécifique d'acide nucléique bicaténaire dans un échantillon, ou pour la distinction entre deux séquences différentes d'acide nucléique bicaténaire dans ledit échantillon, lequel procédé comprend d'abord l'amplification exponentielle de la séquence ou des séquences (si elles existent) spécifiques par les étapes suivantes, et ensuite la détection de la séquence ou des séquences (si elles existent) ainsi amplifiées:

(a) séparation des brins d'acide nucléique dans l'échantillon et traitement de l'échantillon par un excès molaire d'une paire d'amorces oligonucléotidiques pour chaque séquence spécifique différente qui est détectée, une amorce pour chaque brin, dans des conditions d'hybridation et en présence d'un agent inducteur de polymérisation et des différents nucléoside triphosphates de manière à synthétiser pour chacun desdits brins un produit d'extension de l'amorce respective, qui est complémentaire du brin, lesdites amorces étant choisies de manière que chacune soit pratiquement complémentaire d'une extrémité de la séquence à amplifier sur l'un des brins, de sorte que le produit d'extension synthétisé à partir d'une amorce, lorsqu'il est séparé de son complément, peut servir de matrice pour la synthèse d'un produit d'extension de l'autre amorce de la paire;
(b) traitement de l'échantillon résultant de (a), dans des conditions de dénaturation, pour séparer les produits d'extension d'amorces d'avec leurs matrices;
(c) traitement comme en (a) de l'échantillon, résultant de (b), par des amorces oligonucléotidiques de manière à synthétiser un produit d'extension d'amorce au moyen de chacun des monobrins produits dans l'étape (b) en tant que matrice; et, si on le désire,
(d) répétition au moins une fois des étapes (b) et (c); l'amplification exponentielle de la séquence ou des séquences, si elles existent, d'acide nucléique ayant donc pour résultat de permettre leur détection; et, si on le désire,
(e) addition au produit de l'étape (c) ou (d) d'une sonde oligonucléotidique marquée capable d'hybridation avec ladite séquence à détecter; et
(f) détermination ayant pour but d'établir si ladite hybridadation a eu lieu.

**2.** Procédé selon la revendication 1, dans lequel une séquence à amplifier et détecter est contenue dans une séquence plus grande.

**3.** Procédé selon la revendication 1 ou 2, dans lequel l'agent de polymérisation est l'ADN polymérase I de E. coli, le fragment de Klenow de l'ADN polymérase I de E. coli, l'ADN polymérase de T4, la transcriptase inverse ou une enzyme thermostable.

**4.** Procédé selon l'une quelconque des revendications 1 à 3, dans lequel l'acide nucléique est l'ADN et les amorces sont des oligodésoxyribonucléotides.

**5.** Procédé selon l'une quelconque des revendications 1 à 4, dans lequel chaque amorce utilisée contient un site de restriction qui est identique à ou différent d'un site de restriction de l'autre amorce, et, après l'étape (c) ou (d) et avant l'étape (e), le produit de l'étape (c) ou (d) (selon le cas) est coupé par une enzyme de restriction spécifique de chacun desdits sites de restriction et les produits coupés sont séparés d'avec les produits non coupés, et utilisés dans l'étape (e).

**6.** Procédé selon l'une quelconque des revendications 1 à 5, dans lequel une séquence à amplifier et à détecter est associée à une maladie génétique, cancéreuse ou infectieuse.

**7.** Procédé selon l'une quelconque des revendications 1 à 6, dans lequel on traite d'abord une séquence d'acide nucléique monocaténaire que l'on désire détecter, dans des conditions d'hybridation, par une amorce, un agent inducteur de polymérisation et les différents nucléoside triphosphates, pour former un brin complémentaire de celle-ci, pour obtenir ladite séquence d'acide nucléique bicaténaire de départ et permettre la détection de ladite séquence monocaténaire.

**8.** Procédé pour le clonage, dans un vecteur, d'une séquence ou de séquences spécifique(s) d'acide nucléique bicaténaire, contenue(s) dans un acide nucléique ou un mélange d'acides nucléiques, lequel procédé comprend d'abord l'amplification deladite séquence ou desdites séquences, par les étapes suivantes:

(a) séparation des brins d'acide(s) nucléique(s) et traitement de l'acide nucléique ou des acides nucléiques par un excès molaire d'une paire d'amorces oligonucléotidiques pour chaque séquence spécifique distincte qui est amplifiée et clonée, une pour chaque brin, dans des conditions d'hybridation et en présence d'un agent inducteur de polymérisation et des différents nucléoside triphosphates de manière à synthétiser pour chacun desdits brins un produit d'extension de chaque amorce, qui est complémentaire de son brin respectif, lesdites amorces étant choisies de manière que chacune soit pratiquement complémenmentaire d'une extrémité de la séquence à amplifier sur l'un des brins, de sorte que le produit d'extension synthétisé à partir d'une amorce, lorsqu'il est

séparé de son complément, peut servir de matrice pour la synthèse du produit d'extension de l'autre amorce de la paire, et lesdites amorces contenant chacun un site de restriction qui est identique au(x) ou différent du(des) site(s) de restriction de l'autre (des autres) amorce(s);
(b) séparation des produits d'extension d'amorce d'avec les matrices sur lesquelles ils ont été synthétisés, pour la production de molécules monocaténaires;
(c) traitement comme en (a) des molécules monocaténaires, produites dans l'étape (b), par des amorces oligonucléotidiques de manière à synthétiser un produit d'extension d'amorce au moyen de chacun des monobrins produits dans l'étape (b) en tant que matrice; et, si on le désire,
(d) répétition au moins une fois des étapes (b) et (c); l'amplification exponentielle de la séquence ou des séquences d'acide nucléique en résultant;
(e) addition au produit de l'étape (c) ou (d) d'une enzyme de restriction pour chacun des desdits sites de restriction, pour l'obtention de produits coupés dans une digestion de restriction; et
(f) ligature du(des) produit(s) coupé(s) dans un ou plusieurs vecteurs de clonage.

**9.** Procédé pour la synthèse d'un fragment d'acide nucléique à partir d'une séquence existante d'acide nucléique bicaténaire comportant moins de nucléotides que le fragment qui est synthétisé et deux amorces oligonucléotidiques, l'acide nucléique qui est synthétisé étant composé d'un segment gauche, d'un segment médian et d'un segment droit, et le segment médian représentant au moins essentiellement la séquence nucléotidique de ladite séquence existante d'acide nucléique, et les segments droit et gauche représentant la séquence nucléotidique à une extrémité de chacune des deux amorces, dont les autres extrémités sont complémentaires ou pratiquement complémentaires des extrémités des monobrins produits par séparation des brins de ladite séquence existante d'acide nucléique, lequel procédé comprend:
(a) le traitement des brins dudit fragment existant par un excès molaire de deux amorces oligonucléotidiques, une pour chacun des brins, dans des conditions d'hybridation et en présence d'un agent inducteur de polymérisation et des différents nucléoside triphosphates de manière à synthétiser pour chaque brin un produit d'extension de l'amorce respective, qui est complémentaire du brin, lesdites amorces étant choisies de manière que chacune soit pratiquement complémentaire d'une extrémité d'un brin de ladite séquence existante, de sorte que le produit d'extension synthétisé à partir d'une amorce, lorsqu'il est séparé de son complément, peut servir de matrice pour la synthèse d'un produit d'extension de l'autre amorce, et dans lequel chaque amorce contient également une séquence de nucléotides qui ne sont pas complémentaires de ladite séquence existante et qui correspondent aux deux extrémités du fragment d'acide nucléique qui est synthétisé;
(b) la séparation des produits d'extension d'amorce d'avec les matrices sur lesquelles ils ont été synthétisés, pour la production de molécules monocaténaires; et
(c) le traitement des molécules monocaténaires provenant de l'étape (b) par les amorces de l'étape (a), dans des conditions telles qu'un produit d'extension d'amorce est synthétisé au moyen de chacun des monobrins produits dans l'étape (b), en tant qu'amorce, de manière à produire deux molécules intermédiaires d'acide nucléique bicaténaire, dans chacune desquelles a été incorporée la séquence nucléotidique présente à une extrémité des amorces oligonucléotidiques, et deux molécules complètes d'acide nucléique bicaténaire, dans chacune desquelles a été incorporée la séquence nucléotidique à l'extrémité de l'une et l'autre des amorces oligonucléotidiques;
(d) la répétition des étapes (b) et (c) un nombre de fois suffisant pour produire en une quantité suffisante les molécules bicaténaires complètes.

**10.** Procédé selon la revendication 9, dans lequel le produit de l'étape (d) est ensuite utilisé pour la production d'une séquence d'acide nucléique étendue davantage, par extension de celle-ci selon un procédé tel que défini dans la revendication 9.

**11.** Procédé selon la revendication 9 ou 10, dans lequel la séquence existante d'acide nucléique est obtenue par traitement de deux oligonucléotides, dont chacun comporte à l'une de ses extrémités une séquence nucléotidique qui est complémentaire d'une extrémité de l'autre oligonucléotide et qui sont complémentaires l'une de l'autre à leurs autres extrémités, par un agent de polymérisation et les différents nucléoside triphosphates, dans des conditions telles qu'est synthétisé un produit d'extension de chaque oligonucléotide, qui est complémentaire de l'autre des produits d'extension résultants.

**12.** Procédé selon l'une quelconque des revendications 8 à 10, dans lequel on traite d'abord une séquence

d'acide nucléique monocaténaire, dans des conditions d'hybridation, par une amorce, un agent inducteur de polymérisation et les différents nucléoside triphosphates, pour former un brin complémentaire de celui-ci afin obtenir ainsi ladite séquence d'acide nucléique bicaténaire de départ.

**13.** Nécessaire pour la détection d'au moins une séquence spécifique d'acide nucléique dans un échantillon, lequel nécesaire comprend, sous forme emballée, une unité à plusieurs contenants comportant:

(a) chacune de deux amorces oligonucléotidiques pour chaque séquence distincte à détecter,

(I) si la séquence spécifique d'acide nucléique à détecter est monocaténaire, une amorce étant pratiquement complémentaire d'une extrémité du brin, de sorte qu'un produit d'extension deladite amorce, formé dans des conditions d'hybridation et en présence d'un agent inducteur de polymérisation et des différents nucléoside triphosphates, est pratiquement complémentaire dudit brin, et l'autre amorce étant pratiquement complémentaire d'une extrémité du brin dudit produit d'extension et pouvant être utilisée, dans des conditions d'hybridation et en présence d'un agent inducteur de polymérisation et des différents nucléoside triphosphates, pour synthétiser un autre produit d'extension en utilisant comme matrice ledit produit d'extension de de ladite amorce en tant que matrice, afin d'obtenir ainsi un acide nucléique constitué de deux brins; ou

(II) si la séquence spécifique d'acide nucléique à détecter est bicaténaire, les amorces étant telles que chacune est pratiquement complémentaire d'une extrémité de l'un des brins et un produit d'extension synthétisé à partir de chaque amorce, au moyen de son brin complémentaire en tant que matrice, dans des conditions d'hybridation et en présence d'un agent inducteur de polymérisation et des différents nucléoside triphosphates, lorsqu'il est séparé de son complément, pouvant servir de matrice pour la synthèse d'un produit d'extension de l'autre amorce dans des conditions d'hybridation et en présence d'un agent d'induction de polymérisation et des différents nucléoside triphosphates;

(b) un agent de polymérisation;

(c) chacun des différents nucloside triphosphates;

(d) une sonde oligonucléotidique capable d'hybridation avec ladite séquence si celle-ci est présente dans ledit échantillon; et

(e) un moyen pour la détection d'hybrides de ladite sonde et de ladite séquence.

# FIG.1

Double-Stranded 94-bp Sequence

TTTGC TTCTGACACA ACTGTGTTCA CTAGCAACCT →
AAACG AAGACTGTGT TGACACAAGT GATCGTTGGA

NcoI HinfI MstII

CAAACAGACA CCATGGTGCA CCTGACTCCT GAGGAGAAGT →
GTTTGTCTGT GGTACCACGT GGACTGAGGA CTCCTCTTCA

Allelic base pair DNA polymorphism

CTGCCGTTAC TGCCCTGTG
GACGGCAATG ACGGGACAC

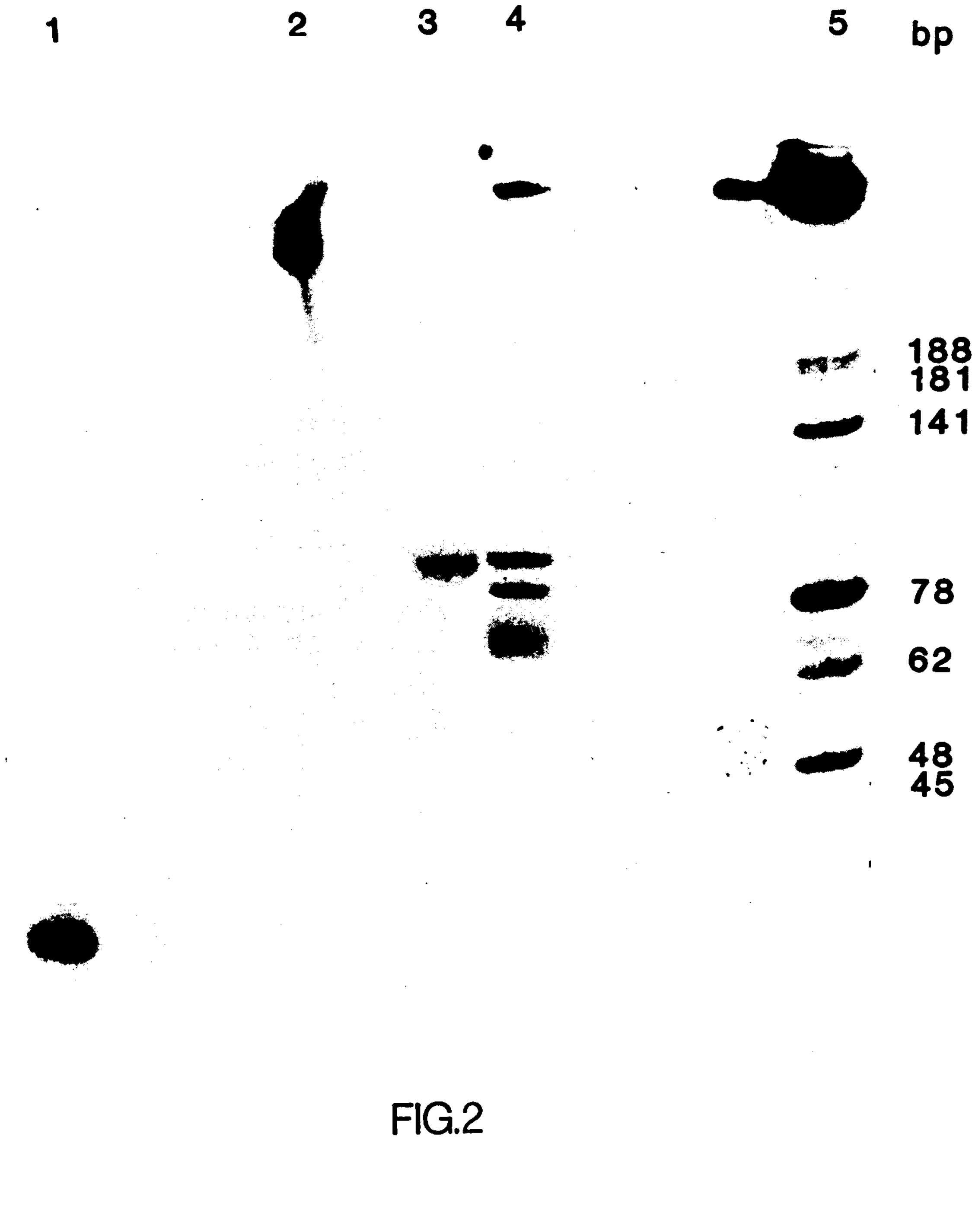


FIG.2

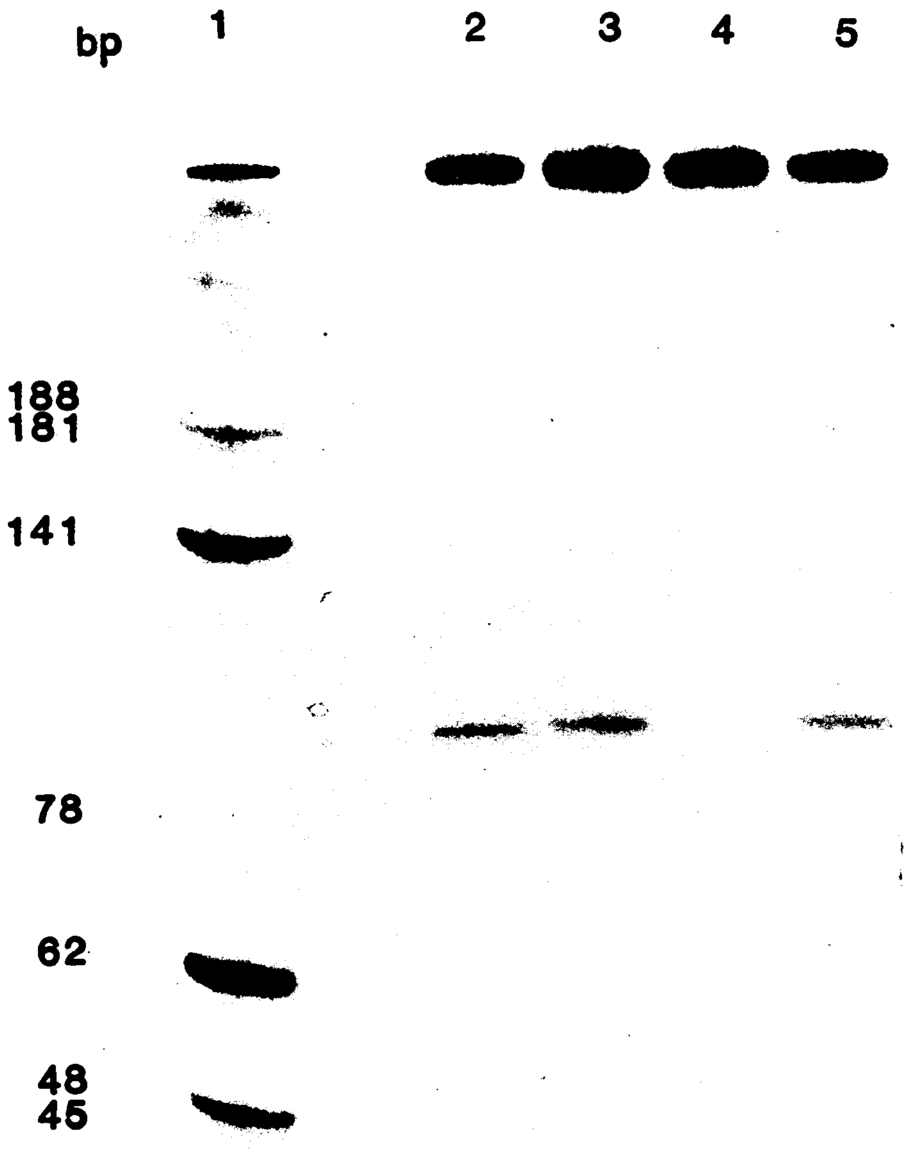


FIG.3

Human Betaglobin FIG.4-1

```
0 ...CCATCTATTG CTTACATTTG CTTCTGACAC AACTGTGTTC ACTAGCAACC ACCTGACTCC TGAGGAGAAG TCTGCCGTTA CTGCCCTGTG GGGCAAGGTG AAGTTGGTGG..
     !          !          !          !          !          !          !          !          !          !          !
     101        111        121        131 ***    161        171        181        191        201        211        221
     !          !          !          !          !          !          !          !          !          !          !
0 ...GGTAGATAAC GAATGTAAAC GAAGACTGTG TTGACACAAG TGATCGTTGG TGGACTGAGG ACTCCTCTTC AGACGGCAAT GACGGGACAC CCCGTTCCAC TTCAACCACC..

             +
     CACAGGGCAGTAACG  PC01
             +
     TTTGCTTCTGACACA  PC02
                                                                                                                              CYCLES
                                                          !
                                                          :  denature, re-anneal
                                                          v

0 ...CCATCTATTG CTTACATTTG CTTCTGACAC AACTGTGTTC ACTAGCAACC ACCTGACTCC TGAGGAGAAG TCTGCCGTTA CTGCCCTGTG GGGCAAGGTG AAGTTGGTGG...
                                                                                  !!!!! !!!!!!!!!!
                                                                 extends <---- GCAAT GACGGGACAC    5'  PC01

           5'   PC02  TTTG CTTCTGACAC A ----> extends
                      !!!! !!!!!!!!!! !
0 ...GGTAGATAAC GAATGTAAAC GAAGACTGTG TTGACACAAG TGATCGTTGG TGGACTGAGG ACTCCTCTTC AGACGGCAAT GACGGGACAC CCCGTTCCAC TTCAACCACC...
                                                                                                                                  1
                                                          !
                                                          !  Polymerase, dNTPs
                                                          v

0 ...CCATCTATTG CTTACATTTG CTTCTGACAC AACTGTGTTC ACTAGCAACC ACCTGACTCC TGAGGAGAAG TCTGCCGTTA CTGCCCTGTG GGGCAAGGTG AAGTTGGTGG...
     !!!!!!!!!! !!!!!!!!!! !!!!!!!!!! !!!!!!!!!! !!!!!!!!!! !!!!!!!!!! !!!!!!!!!! !!!!!!!!!! !!!!!!!!!!
1 ...GGTAGATAAC GAATGTAAAC GAAGACTGTG TTGACACAAG TGATCGTTGG TGGACTGAGG ACTCCTCTTC AGACGGCAAT GACGGGACAC

1                     TTTG CTTCTGACAC AACTGTGTTC ACTAGCAACC ACCTGACTCC TGAGGAGAAG TCTGCCGTTA CTGCCCTGTG GGGCAAGGTG AAGTTGGTGG...
                      !!!! !!!!!!!!!! !!!!!!!!!! !!!!!!!!!! !!!!!!!!!! !!!!!!!!!! !!!!!!!!!! !!!!!!!!!! !!!!!!!!!! !!!!!!!!!!
0 ...GGTAGATAAC GAATGTAAAC GAAGACTGTG TTGACACAAG TGATCGTTGG TGGACTGAGG ACTCCTCTTC AGACGGCAAT GACGGGACAC CCCGTTCCAC TTCAACCACC...

                                                          !
                                                          !  denature, re-anneal
                                                          v

0 ...CCATCTATTG CTTACATTTG CTTCTGACAC AACTGTGTTC ACTAGCAACC ACCTGACTCC TGAGGAGAAG TCTGCCGTTA CTGCCCTGTG GGGCAAGGTG AAGTTGGTGG..
                                                                                  !!!!! !!!!!!!!!!
                                                                 extends <---- GCAAT GACGGGACAC    5'  PC01

           5'   PC02  TTTG CTTCTGACAC A ----> extends
                      !!!! !!!!!!!!!! !
1 ...GGTAGATAAC GAATGTAAAC GAAGACTGTG TTGACACAAG TGATCGTTGG TGGACTGAGG ACTCCTCTTC AGACGGCAAT GACGGGACAC
                                                                                                                                  2
1                     TTTG CTTCTGACAC AACTGTGTTC ACTAGCAACC ACCTGACTCC TGAGGAGAAG TCTGCCGTTA CTGCCCTGTG GGGCAAGGTG AAGTTGGTGG...
                                                                                  !!!!! !!!!!!!!!!
                                                                 extends <---- GCAAT GACGGGACAC    5'  PC01

           5'   PC02  TTTG CTTCTGACAC A ----> extends
                      !!!! !!!!!!!!!! !
0 ...GGTAGATAAC GAATGTAAAC GAAGACTGTG TTGACACAAG TGATCGTTGG TGGACTGAGG ACTCCTCTTC AGACGGCAAT GACGGGACAC CCCGTTCCAC TTCAACCACC...
```

FIG.4-2

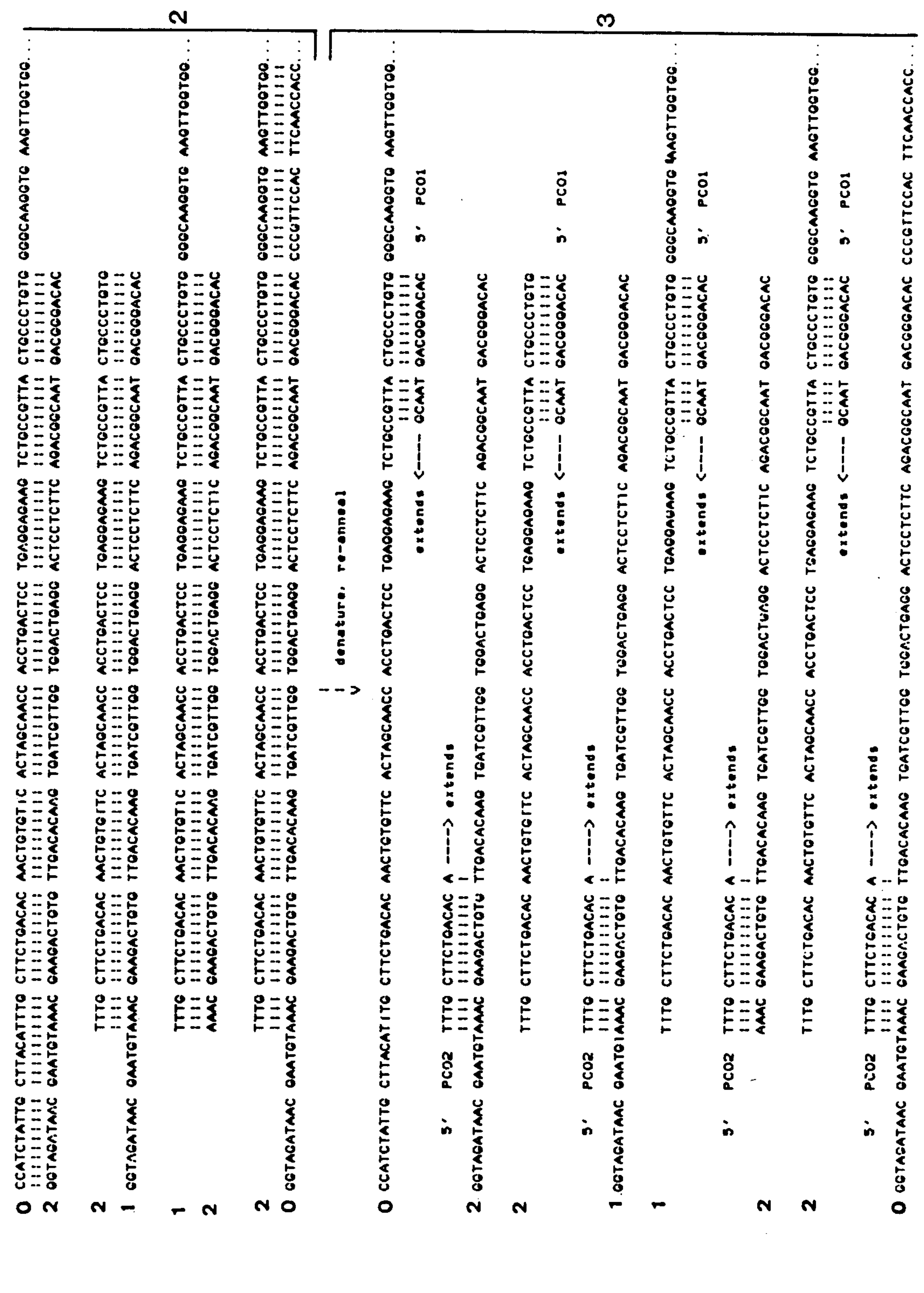

FIG.4-3

```
                                     |
                                     |  Polymerase, dNTPs
                                     v

0 ..CCATCTATTG CTTACATTTG CTTCTGACAC AACTGTGTTC ACTAGCAACC ACCTGACTCC TGAGGAGAAG TCTGCCGTTA CTGCCCTGTG GGGCAAGGTG AAGTTGGTGG...
    :::::::::: :::::::::: :::::::::: :::::::::: :::::::::: :::::::::: :::::::::: :::::::::: ::::::::::
3   GGTAGATAAC GAATGTAAAC GAAGACTGTG TTGACACAAG TGATCGTTGG TGGACTGAGG ACTCCTCTTC AGACGGCAAT GACGGGACAC

3                    TTTG CTTCTGACAC AACTGTGTTC ACTAGCAACC ACCTGACTCC TGAGGAGAAG TCTGCCGTTA CTGCCCTGTG
                     :::: :::::::::: :::::::::: :::::::::: :::::::::: :::::::::: :::::::::: ::::::::::
2 ..GGTAGATAAC GAATGTAAAC GAAGACTGTG TTGACACAAG TGATCGTTGG TGGACTGAGG ACTCCTCTTC AGACGGCAAT GACGGGACAC

2                    TTTG CTTCTGACAC AACTGTGTTC ACTAGCAACC ACCTGACTCC TGAGGAGAAG TCTGCCGTTA CTGCCCTGTG
                     :::: :::::::::: :::::::::: :::::::::: :::::::::: :::::::::: :::::::::: ::::::::::
3                    AAAC GAAGACTGTG TTGACACAAG TGATCGTTGG TGGACTGAGG ACTCCTCTTC AGACGGCAAT GACGGGACAC

3                    TTTG CTTCTGACAC AACTGTGTTC ACTAGCAACC ACCTGACTCC TGAGGAGAAG TCTGCCGTTA CTGCCCTGTG
                     :::: :::::::::: :::::::::: :::::::::: :::::::::: :::::::::: :::::::::: ::::::::::
1   GGTAGATAAC GAATGTAAAC GAAGACTGTG TTGACACAAG TGATCGTTGG TGGACTGAGG ACTCCTCTTC AGACGGCAAT GACGGGACAC

1                    TTTG CTTCTGACAC AACTGTGTTC ACTAGCAACC ACCTGACTCC TGAGGAGAAG TCTGCCGTTA CTGCCCTGTG GGGCAAGGTG AAGTTGGTGG...
                     :::: :::::::::: :::::::::: :::::::::: :::::::::: :::::::::: :::::::::: ::::::::::
3                    AAAC GAAGACTGTG TTGACACAAG TGATCGTTGG TGGACTGAGG ACTCCTCTTC AGACGGCAAT GACGGGACAC

3                    TTTG CTTCTGACAC AACTGTGTTC ACTAGCAACC ACCTGACTCC TGAGGAGAAG TCTGCCGTTA CTGCCCTGTG
                     :::: :::::::::: :::::::::: :::::::::: :::::::::: :::::::::: :::::::::: ::::::::::
2                    AAAC GAAGACTGTG TTGACACAAG TGATCGTTGG TGGACTGAGG ACTCCTCTTC AGACGGCAAT GACGGGACAC

2                    TTTG CTTCTGACAC AACTGTGTTC ACTAGCAACC ACCTGACTCC TGAGGAGAAG TCTGCCGTTA CTGCCCTGTG GGGCAAGGTG AAGTTGGTGG...
                     :::: :::::::::: :::::::::: :::::::::: :::::::::: :::::::::: :::::::::: ::::::::::
3                    AAAC GAAGACTGTG TTGACACAAG TGATCGTTGG TGGACTGAGG ACTCCTCTTC AGACGGCAAT GACGGGACAC

3                    TTTG CTTCTGACAC AACTGTGTTC ACTAGCAACC ACCTGACTCC TGAGGAGAAG TCTGCCGTTA CTGCCCTGTG GGGCAAGGTG AAGTTGGTGG...
                     :::: :::::::::: :::::::::: :::::::::: :::::::::: :::::::::: :::::::::: :::::::::: :::::::::: ::::::::::
0   GGTAGATAAC GAATGTAAAC GAAGACTGTG TTGACACAAG TGATCGTTGG TGGACTGAGG ACTCCTCTTC AGACGGCAAT GACGGGACAC CCCGTTCCAC TTCAACCACC...
```

3

COPIES OF DNA SEQUENCE AFTER N CYCLES

| N | 0 | 1 | 5 | 10 | 15 | 20 |
|---|---|---|---|---|---|---|
| template | 1 | 1 | 1 | 1 | 1 | 1 |
| long product | 0 | 1 | 5 | 10 | 15 | 20 |
| short product =2(expN)-N-1 | 0 | 0 | 26 | 1013 | 32,752 | 1,048,555 |

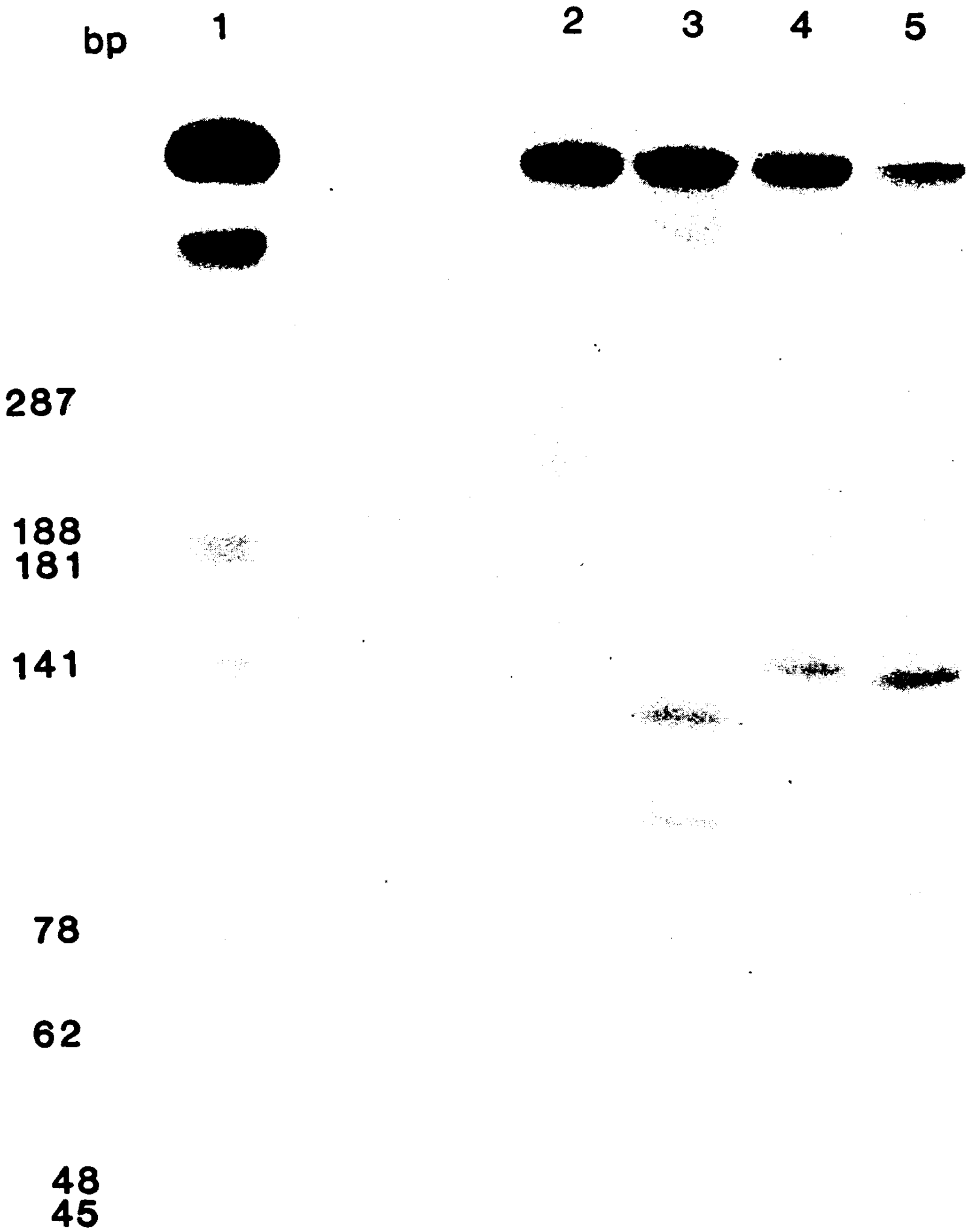


FIG.5

FIG.6

```
                =====-----
βA      CATGGTGCACCTGACTCCTGAGGAGAAGTCTGCCGTTACTGCCCTGTGGGGCAAGGTGAA
        GTACCACGTGGACTGAGGACTCCTCTTCAGACGGCAATGACGGGACACCCCGTTCCACTT
                =====-----

                =====   *
βS      CATGGTGCACCTGACTCCTGTGGAGAAGTCTGCCGTTACTGCCCTGTGGGGCAAGGTGAA
        GTACCACGTGGACTGAGGACACCTCTTCAGACGGCAATGACGGGACACCCCGTTCCACTT
                =====
```

* Marks the mutation (A to T) in the sickle cell gene which disrupts the DdeI site

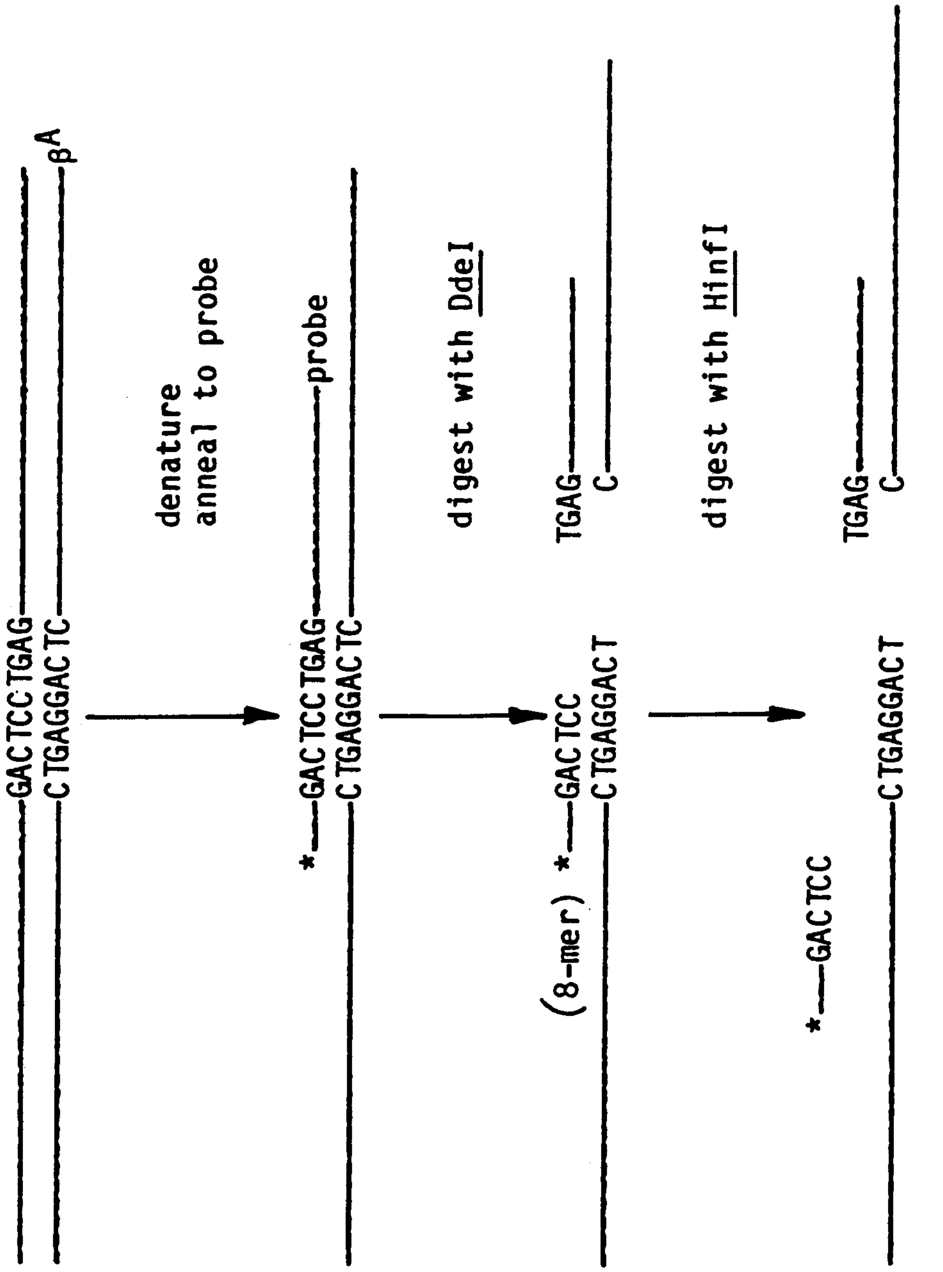


FIG.7

* is label

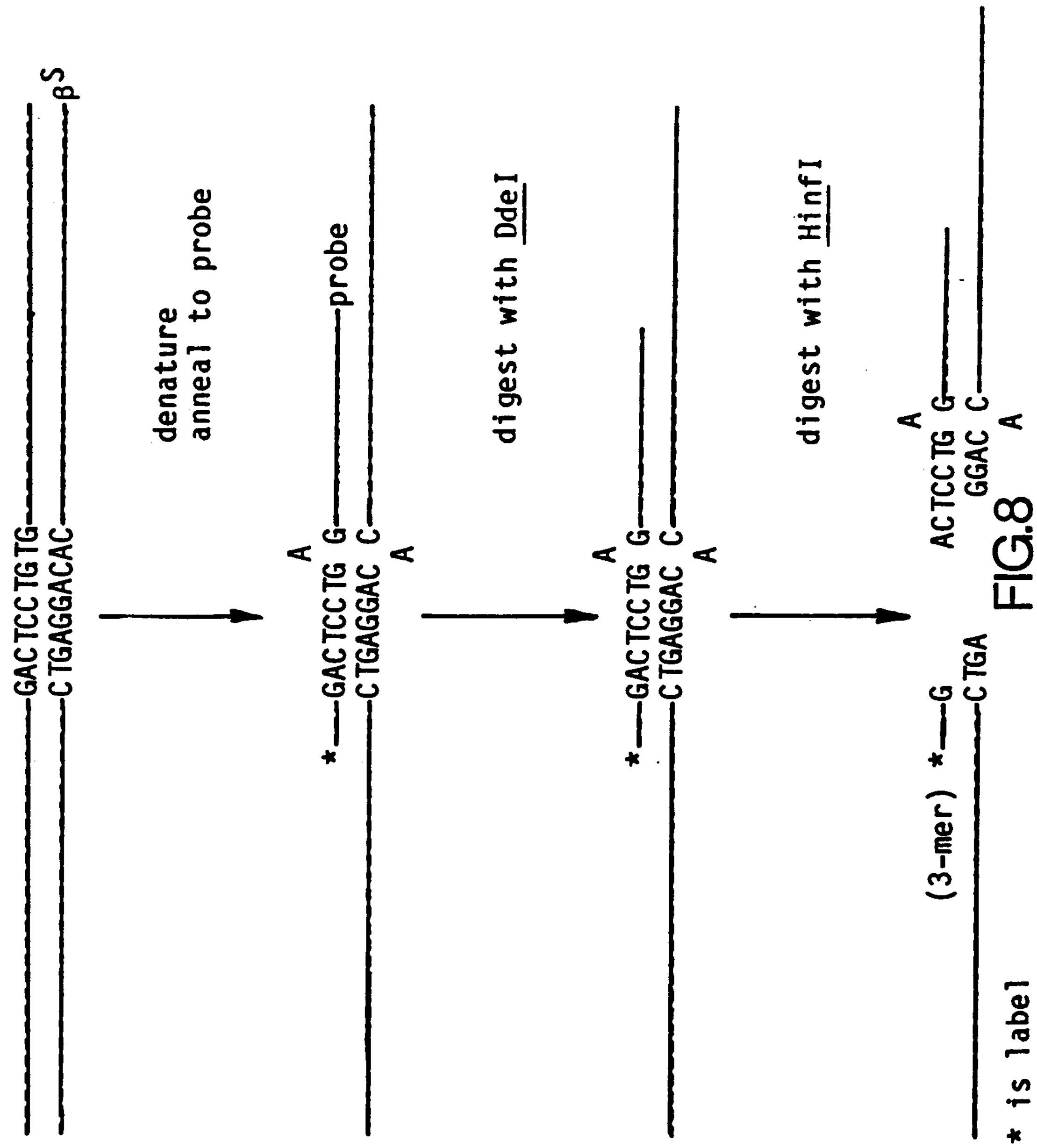
GACTCCTGTG
CTGAGGACAC
β^S
denature
anneal to probe
A
GACTCCTG G
CTGAGGAC C
A
probe
digest with DdeI
A
GACTCCTG G
CTGAGGAC C
A
digest with HinfI
(3-mer)
G
CTGA
A
ACTCCTG G
GGAC C
A
* is label

FIG.8

FIG.9

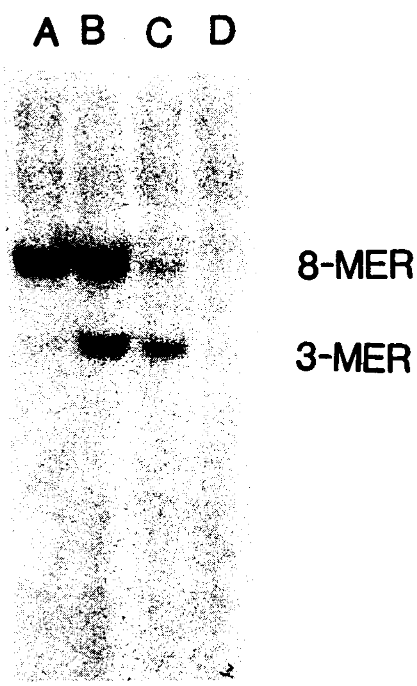

FIG. 10